(19) Europäisches Patentamt
European Patent Office
Office européen des brevets

(11) EP 1 726 313 A1

(12) **EUROPEAN PATENT APPLICATION**
published in accordance with Art. 158(3) EPC

(43) Date of publication:
**29.11.2006 Bulletin 2006/48**

(21) Application number: **05703961.2**

(22) Date of filing: **14.01.2005**

(51) Int Cl.:
***A61K 45/00*** *(2006.01)* ***A61K 48/00*** *(2006.01)*
***A61K 31/7088*** *(2006.01)* ***A61K 39/395*** *(2006.01)*
***A61P 9/10*** *(2006.01)* ***A61P 43/00*** *(2006.01)*
***C12N 15/11*** *(2006.01)* ***C12Q 1/48*** *(2006.01)*
***C12Q 1/68*** *(2006.01)* ***G01N 33/15*** *(2006.01)*
***G01N 33/50*** *(2006.01)* ***G01N 33/53*** *(2006.01)*

(86) International application number:
**PCT/JP2005/000733**

(87) International publication number:
**WO 2005/067971 (28.07.2005 Gazette 2005/30)**

(84) Designated Contracting States:
**AT BE BG CH CY CZ DE DK EE ES FI FR GB GR HU IE IS IT LI LT LU MC NL PL PT RO SE SI SK TR**

(30) Priority: **16.01.2004 JP 2004009383**

(71) Applicant: **Takeda Pharmaceutical Company Limited**
**Osaka-shi,**
**Osaka 541-0045 (JP)**

(72) Inventors:
- **FUSE, Hiromitsu**
**6620084 (JP)**
- **SHIBATA, Sachio**
**3050033 (JP)**
- **TANIYAMA, Yoshio**
**3050821 (JP)**

(74) Representative: **Helbing, Jörg**
**Patentanwälte**
**von Kreisler-Selting-Werner,**
**Postfach 10 22 41**
**50462 Köln (DE)**

# (54) DRUG FOR PREVENTING AND TREATING ARTERIOSCLEROSIS

(57) The present invention relates to a prophylactic/therapeutic agent for atherosclerosis comprising GM3 synthase inhibitor or inhibitor for expression of GM3 synthase gene, a diagnostic product for atherosclerosis comprising antibody to GM3 synthase, a method of diagnosis for atherosclerosis using antibody to GM3 synthase and others, an use of GM3 or GM3 synthase for diagnostic marker of atherosclerosis, and the like.

EP 1 726 313 A1

## Description

### TECHNICAL FIELD

The present invention relates to a medicament for prevention/treatment of atherosclerosis, a diagnostic product for atherosclerosis, a diagnostic marker for atherosclerosis, and others.

### BACKGROUND ART

As the cause of developing the ischemic organ diseases such as ischemic heart disease and cerebrovascular disease which occupies the commonest cause of death, the existence of atherosclerosis is important. Pathomorphismic features of atherosclerotic lesions include fatty streak in subendothelial layer, wherein cholesterol ester is accumulated into cells and the cells which mainly consists of macrophage (foame cells) are illuviate, and fibrous plaque, wherein the status are further proceeded and infiltration of smooth muscle cells, macrophage or T cell, cell necrosis and lipid storage are detected. The region which accumulates lipids shows a structural weakness. In such region, plaque rapture is caused by hemodynamical force, and blood clot is rapidly formed by the reaction of tissue factors with blood coagulation factors. It has been elucidated that plaque rapture in coronary artery and thrombotic occlusion closely relate to the onset of so-called acute coronary syndromes such as acute myocardial infarction, unstable angina, cardiac sudden death (Fuster, V. et al., N.Engl. J. Med., 326, 242-250, 1992).

It is shown that the most important risk factor of acute coronary syndromes is a cholesterol level in blood serum, especially low density lipoprotein-cholesterol (LDL-C) level by several large scale epidemiological investigation (4S study, CARE study and the like). By the evidence that blood plasma hypocholesterolemic treatment by statin-like agent, the HMG-CoA reductase inhibitor reduced a developing ratio of heart diseases, validity of LDL as a risk factor can be demonstrated.

By cell biological investigation, it becomes clear that macrophage cell uptakes oxidative LDL by the receptor called scavenger receptor family, wherein LDL, the major lipid carrier in blood, is modified. This receptor does not receive a negative-feedback by the intracellular cholesterol level, and the macrophage captures excess oxidative LDL through this receptor and becomes a foame cell.

The following has been strongly suggested: in patients with hypercholesteremia hyper-low density lipoproteinemia (hyper-LDL-nemia) is shown and readily-oxidative feature of LDL is reported; recently, quantification system of oxidative LDL in blood using a specific antibody to oxidative LDL has been developed; in a group of patients with high risk of heart disease its blood level is increased, and further cell number of oxidative LDL-positive macrophage are increased at the atherosclerotic lesion (Ehara, S. et al., Circulation, 103, 1955-1960, 2001); formation of atherosclerotic lesion based on the mechanism of macrophage foam cell formation by oxidative LDL occurs in the living body. Oxidative LDL shows, in addition to foaming a macrophage cell, atherosclerosis-inducing function to vascular endothelial cells, smooth muscle cells and macrophage cells. One of the functions includes induction of apoptosis. The present inventors have reported that as for the apoptosis of foaming macrophage, an enzyme involving in the metabolic system of ceramide plays an important role and participates in the formation of atherosclerosis (WO 03/78624 publication).

On the other hand, accumulation of sphingoglycolipids having a ceramide as a hydrophobic group in the atherosclerototic lesion has been reported (Arterioscler Thromb Vasc Biol., 15, 1607-1615, 1995). It becomes obvious that, among them, accumulation of GM3 in WHHL rabbit is increased by 11-fold compared to that in normal rabbit (Hara, A. and Taketomi, T., J. Biochem. 109, 904-908, 1991), and in human aorta lesion, anti-GM3 antibody-positive cells are accumulated (Bobryshev, Y.V., et al, Biochim. Biophys. Acta, 1535, 87-99, 2001). Moreover, there is also a report that GM3 accelerates uptake of LDL by macrophage (Prokazova N et al., Biochem Biophys. Res. Commun. 177, 582-587, 1991). However, at present, there is no idea whether the accumulation mechanism of GM3 and further the accumulation are related to the atherosclerotic lesion formation at all.

As for the pathway which generates GM3 in vivo, two kinds of pathways are mainly known. One is a pathway, in which N-acetylneuraminic acid is added to lactosylceramide to synthesize GM3, and is catalyzed by GM3 synthase (Kolter, T. et al., J. Biol. Chem., 277, 25859-25862, 2002). The other is a pathway, in which N-acetylglucosamine is removed from GM2 to synthesize GM3, and is catalyzed by $\beta$-hexosaminidase (Tettamanti, G. et al., Biochimie, 85, 423-437, 2003).

Also, it has been reported there is a possibility that GM3 is increased by TNF$\alpha$ stimulus and becomes the cause substance for the insulin resistance (Tagami, S. et al., J. Biol. Chem., 277, 3085-3092, 2002). Since, in the case where GM3 synthase was overexpressed in 3T3-L1 adipose cells, autophosphorylation of insulin receptor is not changed, and selective suppression of phosphorylation of IRS-1, phosphorylation of IRS-1 by insulin stimulus and/or suppression of uptake of glucose in the case where GM3 is added to adipose tissue occur, it is indicated that there is a possibility that GM3 itself induces insulin resistance.

Also, mRNA encoding GM3 synthase from the animal, in which TNF$\alpha$ is overexpressed in adipose tissue, such as Zucker fa/fa rat, ob/ob mouse and others is remarkably high compared to that from the corresponding lean animal. From these results, it is speculated that in adipose tissue, by TNFα signal, intracellular GM3 is increased, and internalization of insulin receptor is suppressed to appear insulin resistance.

GM3 synthase is a Type II membrane protein having a transmembrane region at the N-terminus and existing in Golgi lumen. It is said that GM3 synthase highly expresses in brain, skeletal muscle, testis and others (Ishii, A. et al., J. Biol. Chem., 273, 31652-31655, 1998). However, since GM3 synthase-deficient mouse grows to adult animal (Yamashita, T. et al., Proc. Natl. Acad. Sci. USA, 100, 3445-3449, 2003), it is indicated that GM3 synthase activity is not essential for growth of mouse. This deficient mouse is highly susceptible to insulin and functions defensively to insulin resistance of fatty food loading. Thus it may be considered that GM3 is a negative controlling factor to insulin signal.

## DISCLOSURE OF THE INVENTION

It is longed for targeting a molecule, in which its expression is specifically varied in a developing model animal for atherosclerosis, establishing a useful diagnostic marker, elucidating dynamics of protein in blood plasma in atherosclerosis, and further providing a superior medicament having prophylactic/therapeutic effects on atherosclerosis by a mechanism different from controlling a risk factor of atherosclerosis historically known.

As a result of extensive investigations, the present inventors have found that the expression of GM3 synthase is induced by atherosclerosis-inducing stimulus and GM3 synthase has an atherosclerosis-enhancing activity. That is, among the genes, in which its expression is increased in aorta sample of ApoE-knockout mouse, a developing model animal for atherosclerosis, an increased metabolism of glycosphingolipid (GSL) was detected by the pathway analysis to determine a change of signal transduction system or whole metabolic system. Among them, it was found that the expression of GM3 synthase, wherein its accumulation at atherosclerotic lesions in human or rabbit is reported, is significantly increased in macrophage cells by atherosclerosis-inducing stimulus. In macrophage existing at atherosclerotic lesions, an intracellular GM3 is increased by atherosclerosis-inducing stimulus including INF-γ, thereby reduction of cholesterol transporting ability and/or induction of inflammatory reaction, MMP producing ability and others occur. As a result, it is thought that GM3 functions as atherosclerosis enhancement. By elucidating an atherosclerosis enhancing function of GM3 synthase and inhibiting the function efficiently, a novel method of preventing/treating atherosclerosis can be established.

Based on these findings, the present inventors have contemplated for a possibility that the medicament that inhibits GM3 synthase may be a prophylactic/therapeutic agent for atherosclerosis as well as an improving drug for insulin resistance, and have continued further extensive studies and as a result, have come to accomplish the present invention.

That is, the present invention provides:

(1) A medicament for prevention/treatment of atherosclerosis comprising a GM3 synthase inhibitor;

(2) A medicament for prevention/treatment of atherosclerosis comprising an inhibitor for expression of GM3 synthase gene;

(3) The medicament of (1) or (2), wherein GM3 synthase is a protein comprising the same or substantially the same amino acid sequence as that of SEQ ID NO: 1, its partial peptide or a salt thereof;

(4) The medicament of (1) or (2), wherein GM3 synthase is a protein consisting of the amino acid sequence of SEQ ID NO: 1, or a salt thereof;

(5) An antisense polynucleotide comprising a base sequence complement to or substantially complement to that of the polynucleotide coding for the protein having the same or substantially the same amino acid sequence as that of SEQ ID NO: 1, or a partial peptide thereof;

(6) A medicament comprising the antisense polynucleotide of (5);

(7) The medicament of (6), which is a medicament for prevention/treatment of atherosclerosis;

(8) An siRNA or shRNA to the polynucleotide coding for the protein having the same or substantially the same amino acid sequence as that of SEQ ID NO: 1, or a partial peptide thereof;

(9) A medicament comprising the siRNA or shRNA of (8);

(10) The medicament of (9), which is a medicament for prevention/treatment of atherosclerosis;

(11) A medicament for prevention/treatment of atherosclerosis comprising an antibody to GM3 synthase;

(11a) The medicament of (11), wherein the antibody is an antibody to the protein comprising the same or substantially the same amino acid sequence as that of SEQ ID NO: 1, its partial peptide or a salt thereof;

(12) A diagnostic product for atherosclerosis comprising an antibody to GM3 synthase;

(12a) The diagnostic product of (12), wherein the antibody is an antibody to the protein comprising the same or substantially the same amino acid sequence as that of SEQ ID NO: 1, its partial peptide or a salt thereof;

(13) A diagnostic product for atherosclerosis comprising a polynucleotide coding for the protein having the same or substantially the same amino acid sequence as that of SEQ ID NO: 1, or a partial peptide thereof;

(14) A method of diagnosing atherosclerosis which is characterized by using an antibody to GM3 synthase or a polynucleotide coding for the protein having the same or substantially the same amino acid sequence as that of SEQ ID NO: 1, or a partial peptide thereof;
(15) Use of antibody to GM3 synthase or a polynucleotide coding for the protein having the same or substantially the same amino acid sequence as that of SEQ ID NO: 1, or a partial peptide thereof, for manufacturing a diagnostic product for atherosclerosis;
(16) A method of diagnosing atherosclerosis which is characterized by quantifying GM3 in blood plasma of mammal;
(17) Use of GM3 for diagnostic marker of atherosclerosis;
(18) A method of diagnosing atherosclerosis which is characterized by quantifying GM3 synthase in blood plasma of mammal;
(18a) A method of diagnosing atherosclerosis which is characterized by quantifying soluble GM3 synthase in blood plasma of mammal;
(19) Use of GM3 synthase for diagnostic marker of atherosclerosis;
(19a) Use of soluble GM3 synthase for diagnostic marker of atherosclerosis;
(20) A method of screening a prophylactic/therapeutic agent for atherosclerosis which is characterized by using the protein having the same or substantially the same amino acid sequence as that of SEQ ID NO: 1, its partial peptide or a salt thereof;
(21) A kit for screening a prophylactic/therapeutic agent for atherosclerosis which is characterized by comprising the protein having the same or substantially the same amino acid sequence as that of SEQ ID NO: 1, its partial peptide or a salt thereof;
(22) A method of screening a prophylactic/therapeutic agent for atherosclerosis which is characterized by using a polynucleotide coding for the protein having the same or substantially the same amino acid sequence as that of SEQ ID NO: 1, or a partial peptide thereof;
(23) A kit for screening a prophylactic/therapeutic agent for atherosclerosis which is characterized by comprising a polynucleotide coding for the protein having the same or substantially the same amino acid sequence as that of SEQ ID NO: 1, or a partial peptide thereof;
(24) A method of screening a prophylactic/therapeutic agent for atherosclerosis which is characterized by assaying an activity of the protein comprising the same or substantially the same amino acid sequence as that of SEQ ID NO: 1, its partial peptide or a salt thereof;
(25) A method of screening a prophylactic/therapeutic agent for atherosclerosis which is characterized by quantifying the protein comprising the same or substantially the same amino acid sequence as that of SEQ ID NO: 1, its partial peptide or a salt thereof;
(26) A method of quantifying the protein comprising the same or substantially the same amino acid sequence as that of SEQ ID NO: 1, its partial peptide or a salt thereof, which is characterized by using an antibody to GM3 synthase;
(27) A method of screening a prophylactic/therapeutic agent for atherosclerosis which is characterized by quantifying a polynucleotide coding for the protein having the same or substantially the same amino acid sequence as that of SEQ ID NO: 1, or a partial peptide thereof;
(28) A method for preventing/treating atherosclerosis which is characterized by inhibiting GM3 synthase;
(28a) A method for preventing/treating atherosclerosis which is characterized by inhibiting GM3 synthase activity;
(28b) A method for preventing/treating atherosclerosis which is characterized by inhibiting expression or production of GM3 synthase;
(29) A method for preventing/treating atherosclerosis which is characterized by inhibiting an expression of GM3 synthase;
(30) A method for preventing/treating atherosclerosis which is characterized by administering an effective amount of GM3 synthase inhibitor to mammals;
(31) A method for preventing/treating atherosclerosis which is characterized by administering an effective amount of inhibitor for expression of GM3 synthase to mammals;
(32) Use of GM3 synthase inhibitor for manufacturing a prophylactic/therapeutic agent for atherosclerosis; and
(33) Use of inhibitor for expression of GM3 synthase for manufacturing a prophylactic/therapeutic agent for atherosclerosis.

## BEST MODE FOR CARRYING OUT THE INVENTION

GM3 synthase (Ganglioside GM3 synthase ; EC 2.4.99.9) used in the present invention is also called such as SAT-I (sialyltransferase I), or CMP-NeuAc:lactosylceramide alpha-2,3-sialyltransferase, which catalyzes a reaction to synthesize GM3 ($\alpha$-N-acetylneuraminyl-2,3-$\beta$-D-galactosyl-1,4-$\beta$-D-glucosylceramide) which is biosynthesized by the transfer of sialic acid derived from GMP-sialic acid (CMP-N-acetylneuraminate) to non-reduced terminal galactose residue of $\beta$-D-galactosyl-1,4-$\beta$-D-glucosylceramide with $\alpha$-2,3 bond.

GM3 synthase used in the present invention includes, for example, a protein comprising the same or substantially the same amino acid sequence as the amino acid sequence represented by SEQ ID NO: 1 (hereinafter the protein is reffered to as the protein of the present invention or the protein used in the psetent invention). GM3 synthase used in the present invention may be a protein derived from any cells (e.g., liver cells, splenocytes, nerve cells, glial cells, β cells of pancreas, bone marrow cells, mesangial cells, Langerhans' cells, epidermic cells, epithelial cells, goblet cells, endothelial cells, smooth muscular cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), or any tissues where such cells are present, e.g., brain or any region of the brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc. from human and other warm-blooded animals (e.g., guinea pigs, rats, mice, checkens, rabbits, swine, sheep, bovine, monkeys, etc.), may also be a synthetic protein.

Substantially the same amino acid sequence as that represented by SEQ ID NO: 1 includes an amino acid sequence having at least about 50% homology, preferably at least about 60% homology, more preferably at least about 70% homology, furthermore preferably at least about 80% homology, especially preferably at least about 90% homology, most preferably at least about 95% homology, to the amino acid sequence represented by SEQ ID NO: 1. The homology among the amino acid sequences can be calculated using homology calculation algorism NCBI BLAST (National Center for Biotechnology Information Basic Local Alignment Search Tool) under the following conditions: expected value = 10; gap is allowable; matrix = BLOSUM62; filtering = OFF.

Preferable examples of the protein which contains substantially the same amino acid sequence as that represented by SEQ ID NO: 1 include a protein having substantially the same amino acid sequence as that shown by SEQ ID NO: 1 described above and having the activity substantially equivalent to the amino acid sequence represented by SEQ ID NO: 1, etc.

Examples of the protein comprising substantially the same amino acid sequence as that represented by SEQ ID NO: 1 include a protein comprising the amino acid sequence represented by SEQ ID NO: 1, etc. The protein consisting of the amino acid sequence represented by SEQ ID NO: 1 is preferably used.

Examples of the substantially equivalent activity include a enzymatic activity catalyzing a reaction to synthesize GM3 (GM3 synthase activity), etc. The term "substantially equivalent" is used to mean that the nature of the activity is the same (for example, physiologically or pharmacologically). Therefore, although it is preferred that activities such as the GM3 synthase activities, etc. be equivalent (e.g., about 0.01- to about 100-fold, preferably about 0.1- to about 10-fold, more preferably about 0.5- to about 2-fold), quantitative factors such as a level of the activity, a molecular weight of the protein, etc. may differ.

GM3 synthase activity can be determined according to a publicly known method, for example, by the method to quantify GM3 produced by the enzymatic reaction after incubating CMP-sialyc acid (10 to 100 nmol) and the enzyme for a given period of time at 37 °C using lactosyl ceramide as a substrate (e.g., the method described in Methods in Enzymology, 311, 82-94 (2000), etc.), or its modified method.

In addition, GM3 synthase activity can be determined by using, for example, an amout of GM3 which exists on cell surfaces or exists in cells.

Specifically, a method to detect GM3 existing on cell surfaces or in cells of a cell line which expresses GM3 synthase (e.g., B16-F1 murine melanoma cell line, etc.) quantitatively using anti-GM3 antibody is known. A method detecting GM3 existing cell surfaces by chemical luminescence system or chemical coloring system after fixation using formaldehyde, reacting with anti-GM3 antibody (e.g., Clone M2590, etc.), and reacting with HRP-labeled anti-mouse IgM antibody or AP-labeled anti-mouse IgM antibody is known (Wang, X.Q. et al., J. Biol. Chem., 277, 47028-47034, 2002). Furthermore, a method to analyze it using FACS after reacting cells, which are cells fixed with such as formaldehyde or unfixed cells in suspension, with anti-GM3 antibody (e.g., Clone M2590, etc.), and reacting FITC-labeled anti-mouse IgM antibody, etc. is known (Tagami, S. et al., J. Biol. Chem., 277, 3085-3092, 2002). Besides, GM3 amount in cells are become to be detectable by lysing cells by Triton X-100, etc. (Inokuchi, J. et al., J. Cell Phys., 141, 573-583, 1989).

The term "inhibition of GM3 synthase" in the present specification may be either inhibition of the activity of the GM3 synthase or inhibition of production of the GM3 synthase (preferably, inhibition of the activity of the GM3 synthase).

Further, the term "GM3 synthase inhibitor" in the present specification may be either a compound or its salt which inhibits the activity of the protein comprising the same or substantially the same amino acid sequence as the amino acid sequence represented by SEQ ID NO: 1 or its partial peptide or its salt; a compound or its salt which inhibits production of the protein comprising the same or substantially the same amino acid sequence as the amino acid sequence represented by SEQ ID NO: 1 or its partial peptide or its salt (preferably, a compound or its salt which inhibits the activity of the protein comprising the same or substantially the same amino acid sequence as the amino acid sequence represented by SEQ ID NO: 1 or its partial peptide or its salt). A compound or its salt which inhibit the expression of the polynulceotide encoding a protein comprising the same or substantially the same amino acid sequence as the amino acid sequence represented by SEQ ID NO: 1 or its partial peptide is used as "an inhibitor of the expression of the gene of GM3 synthase" in the present specification.

Proteins used in the present invention include, for example, so called muteins, such as proteins comprising (1) (i) amino acid sequences represented by SEQ ID NO: 1, wherein at least 1 or 2 amino acids (for example approximately 1 to 100 amino acids, preferably approximately 1 to 30 amino acids, preferably approximately 1 to 10 amino acids, more preferably several (1 to 5) amino acids) are deleted; (ii) amino acid sequences represented by SEQ ID NO: 1, to which at least 1 or 2 amino acids (for example approximately 1 to 100 amino acids, preferably approximately 1 to 30 amino acids, preferably approximately 1 to 10 amino acids, and more preferably several (1 to 5) amino acids) are added; (iii) amino acid sequences represented by SEQ ID NO: 1, into which at least 1 or 2 amino acids (for example approximately 1 to 100 amino acids, preferably approximately 1 to 30 amino acids, preferably approximately 1 to 10 amino acids, and more preferably several (1 to 5) amino acids) are inserted; (iv) amino acid sequences represented by SEQ ID NO: 1, in which at least 1 or 2 amino acids (for example approximately 1 to 100 amino acids, preferably approximately 1 to 30 amino acids, preferably approximately 1 to 10 amino acids, and more preferably several (1 to 5) amino acids) are substituted by other amino acids; or (v) combination of the amino acid sequences described in the above.

When the amino acid sequences have undergone insertion, deletion or substitution as described above, the position of the insertion, deletion or substitution is not particularly limited.

The proteins used in the present invention are represented in accordance with the conventional way of describing peptides, that is, the N-terminus (amino terminus) at the left hand and the C-terminus (carboxyl terminus) at the right hand. In the proteins used in the present invention including the proteins comprising the amino acid sequence represented by SEQ ID NO: 1, the C-terminus may be either a carboxyl group (-COOH), a carboxylate ($-COO^-$), an amide ($-CONH_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl group such as a phenyl-$C_{1-2}$-alkyl group, e.g., benzyl, phenethyl, etc., or an α-naphthyl-$C_{1-2}$-alkyl group such as α-naphthylmethyl, etc.; a pivalivaloyloxymethyl group; and the like.

Where the protein used in the present invention contains a carboxyl group (or a carboxylate) at a position other than the C-terminus, it may be amidated or esterified and such an amide or ester is also included within the protein used in the present invention. The ester group may be the same group as that described with respect to the C-terminus described above.

Furthermore, examples of the protein used in the present invention include variants of the above proteins, wherein the amino group at the N-terminal amino acid residue (for example, a methionine residue) of the protein supra is protected with a protecting group (for example, a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as glycoproteins having sugar chains bound thereto.

Specific examples of the protein used in the present invention include a protein comprising an amino acid sequence represented by SEQ ID NO: 1, etc.

Partial peptides of the protein used in the present invention may be any partial peptides of the protein used in the present invention described above, and preferably, those having properties similar to those of the protein of the present invention described above.

Specifically, for the purpose of preparing the antibody of the present invention described later, peptides having at least 20, preferably at least 50, more preferably at least 70, still more preferably at least 100, and most preferably at least 200 amino acids in the amino acid sequence represented by SEQ ID NO: 1 are used.

The partial peptide used in the present invention may contain an amino acid sequence, wherein at least 1 or 2 amino acids (preferably approximately 1 to 10 amino acids, more preferably several (1 to 5) amino acids) are deleted; to which at least 1 or 2 amino acids (preferably approximately 1 to 20 amino acids, more preferably approximately 1 to 10 amino acids, and most preferably several (1 to 5) amino acids) are added; into which at least 1 or 2 amino acids (preferably about 1 to 20 amino acids, more preferably 1 to 10 amino acids, still more preferably several (1 to 5) amino acids) are inserted; or, in which at least 1 or 2 amino acids (preferably approximately 1 to 10 amino acids, more preferably several and most preferably approximately 1 to 5 amino acids) are substituted by other amino acids.

The partial peptide used in the present invention, similar to the protein used in the present invention described above, includes those having a carboxyl group (or a carboxylate) at a position other than the C-terminus, those wherein an amino group of the N-terminal amino acid residue (e.g., methionine residue) is protected with a protecting group, those wherein the N-terminal region is cleaved in vivo and a glutamine reissue thus formed is pyroglutaminated, those wherein a substituent on the side chain of an amino acid in the molecule is protected with a suitable protecting group, or conjugated proteins such as glycoproteins having sugar chains bound thereto.

The partial peptide used in the present invention can also be used as an antigen for preparing an antibody.

For salts of the protein or the partial peptide used in the present invention, salts with such as physiologically acceptable acids (e.g., inorganic acids or organic acids) or bases (e.g., alkali metal salts) are used, especially physiologically acceptable acid addition salts are preferred. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured from human and other mammalian cells or tissues described above by a publicly known protein purification method, or by culturing a transformant that comprises the DNA encoding the protein. Furthermore, it may also be manufactured according to a peptide synthesis method described hereinafter.

Where it is manufactured from human or other mammalian tissues or cells, human or other mammalian tissues or cells are homogenized, then extracted with an acid or the like, and the extract obtained is isolated and purified by a combination of chromatography techniques such as reverse phase chromatography, ion exchange chromatography, and the like.

To synthesize the protein used in the present invention, its partial peptide, or salts or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc.

Using these resins, amino acids in which $\alpha$-amino groups and functional groups on the side chains are appropriately protected are condensed on the resin in the order of the sequence of the objective protein according to various condensation methods publicly known in the art.

At the end of the reaction, the protein is cut out from the resin and at the same time, the protecting groups are removed. Then, intramolecular disulfide bond-forming reaction is performed in a highly diluted solution to obtain the objective protein or peptide, or amides thereof.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N, N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C1-6) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein or the partial peptide, for example, the α-carboxyl group of the carboxy terminal amino acid is first protected by amidation; the peptide (protein) chain is then extended from the amino group side to a desired length. Thereafter, a protein or partial peptide in which only the protecting group of the N-terminal α-amino group in the peptide chain has been eliminated from the protein and a protein or partial peptide in which only the protecting group of the C-terminal carboxyl group has been eliminated are prepared. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedure similar to the preparation of the amidated protein or peptide above to give the ester form of the desired protein or peptide.

The partial peptide or its salts used in the present invention can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the protein used in the present invention are condensed with the remaining part. Where the product contains protecting groups, these protecting groups are removed to give the desired peptide. Publicly known methods for condensation and elimination of the protecting groups are described in (i) - (v) below.

(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the peptide can be converted into an appropriate salt by a publicly known method; when the protein is obtained in a salt form, it can be converted into a free form by a publicly known method.

The polynucleotide encoding the protein used in the present invention may be any polynucleotide so long as it contains the base sequence encoding the protein used in the present invention described above. The polynucleotide is preferably DNA. The DNA may be any of genomic DNA, genomic DNA library, cDNA derived from the cells and tissues described above, cDNA library derived from the cells and tissues described above and synthetic DNA.

The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

The DNA encoding the protein used in the present invention may be any DNA insofar as it is DNA comprising the base sequence represented by SEQ ID NO: 2, etc. (NM_003896), or DNA comprising the base sequence hybridizable to the base sequence represented by SEQ ID NO: 2, etc., under highly stringent conditions and encoding a protein having the activities substantially equivalent to those of the protein used in the present invention.

Examples of the DNA hybridizable to the base sequence represented by SEQ ID NO: 2 under highly stringent conditions include DNA comprising a base sequence having at least about 50% homology, preferably at least about 60% homology, still more preferably at least about 70% homology, furthermore preferably at least about 80% homology and especially preferably at least about 95% homology, to the base sequence represented by SEQ ID NO: 2, etc.. The homology among the nucleotide sequences can be calculated using homology calculation algorism NCBI BLAST (National Center for Biotechnology Information Basic Local Alignment Search Tool) under the following conditions: expected value = 10; gap is allowable; filtering = ON; match score =1; mismatch score = -3.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

More specifically, for the DNA encoding the protein comprising the amino acid sequence represented by SEQ ID NO: 1, there may be employed DNA comprising the base sequence represented by SEQ ID NO: 2.

The DNA encoding the partial peptide used in the present invention may be any DNA so long as it contains the base sequence encoding the partial peptide used in the present invention described above. The DNA may also be any of genomic DNA, genomic DNA library, cDNA derived from the cells and tissues described above, cDNA library derived from the cells and tissues described above and synthetic DNA.

As the DNA encoding the partial peptide of the present invention, for example, DNA containing a partial base sequence of the DNA comprising the base sequence represented by SEQ ID NO: 2, or DNA comprising a partial base sequence of the DNA comprising a base sequence hybridizable to the DNA containing a base sequence represented by SEQ ID NO: 2, etc., under highly stringent conditions and encoding a protein which has the activities substantially equivalent to those of the protein of the present invention.

The DNA hybridizable with the nucleotide sequence represented by SEQ ID NO: 2, etc., has the same meaning as described above.

As the hybridization method and high stringent conditions, those described above are used.

For cloning of the DNA that completely encodes the protein used in the present invention or its partial peptide (hereinafter sometimes collectively referred to as the protein of the present invention), the DNA may be either amplified by PCR using synthetic DNA primers containing a part of the base sequence of DNA encoding the peptide of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the ODA-LA PCR method, the Gupped duplex method or the Kunkel method or its modification using a publicly known kit, for example, Mutan™-G or Mutan™-K (both manufactured by Takara Shuzo Co., Ltd.).

The cloned DNA encoding the protein can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA containing the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc. Among them, CMV (cytomegalovirus) promoter or SRα promoter is preferably used. Where the host is bacteria of the genus Escherichia, preferred examples of the promoter include trp promoter, lac promoter, recA promoter, $\lambda P_L$ promoter, 1pp promoter, T7 promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker in dhfr gene-deficient Chinese hamster's cells, selection can also be made on thymidine free media.

If necessary and desired, a signal sequence that matches with a host is added to the N-terminus of the protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, $AH22R^-$, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc. Examples of insect cells include, for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977). As the insect, for example, a larva of Bombyx mori can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as $CHO(dhfr^-)$ cell), mouse L cells, mouse AtT-20, mouse myeloma cells, mouse ATDC cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant transformed with the expression vector comprising the DNA encoding the protein can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Journal of Experiments in Molecular Genetics, 431-433, Cold Spring Harbor Laboratory, New York, 1972 ). If necessary and desired, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the protein of the present invention can be produced into the cell, in the cell membrane or outside of the cell of the transformant.

The protein of the present invention can be separated and purified from the culture described above by the following procedures.

When the protein of the present invention is extracted from the culture or cells, after cultivation the transformants or cells are collected by a publicly known method and suspended in a appropriate buffer. The transformants or cells are then disrupted by publicly known methods such as ultrasonication, a treatment with lysozyme and/or freeze-thaw cycling, followed by centrifugation, filtration, etc. Thus, the crude extract of the protein of the present invention can be obtained. The buffer used for the procedures may contain a protein modifier such as urea or guanidine hydrochloride, or a surfactant such as Triton X-100™, etc. When the protein is secreted in the culture, after completion of the cultivation the supernatant can be separated from the transformants or cells to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, it can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein modifying enzyme so that the protein can be appropriately modified to partially remove a polypeptide. Examples of the protein-modifying enzyme include trypsin, chymotrypsin, arginyl endopeptidase, protein kinase, glycosidase or the like.

The presence of the thus formed protein of the present invention can be determined by an enzyme immunoassay or Western blotting using a specific antibody.

Antibodies to the protein of the present invention, its partial peptides, or salts thereof may be any of polyclonal antibodies and monoclonal antibodies, as long as they are capable of recognizing the protein of the present invention, its partial peptides, or salts thereof.

The antibodies to the protein of the present invention, its partial peptides, or salts thereof (hereinafter sometimes merely referred to as the protein of the present invention) may be manufactured by publicly known methods for manufacturing antibodies or antisera, using as antigens the protein of the present invention.

[Preparation of monoclonal antibody]

(a) Preparation of monoclonal antibody-producing cells

The protein of the present invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep, goats and chickens, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the protein, which will be described later, with the antiserum followed by assaying the binding activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., of which PEG is preferably employed.

Examples of the myeloma cells include myeloma cells of warm-blooded animals, such as NS-1, P3U1, SP2/0, AP-1, etc., among which P3U1 is particularly preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the protein etc. as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% $CO_2$. The antibody titer of the culture supernatant of hybridomas can be determined as in the assay for the antibody titer in antisera described above.

(b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

[Preparation of polyclonal antibody]

The polyclonal antibody of the present invention can be manufactured by publicly known methods or modifications thereof. For example, a complex of immunogen (antigen such as the protein of the present invention) and a carrier protein is prepared, and a warm-blooded animal is immunized with the complex in a manner similar to the method described above for the manufacture of monoclonal antibodies. The product containing the antibody to the protein of the present invention is collected from the immunized animal followed by separation and purification of the antibody.
In the complex of an immunogen and a carrier protein used to immunize a warm-blooded mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, hemocyanin, etc. is coupled to hapten in a carrier-to-hapten weight ratio of approximately 0.1 to 20, preferably about 1 to about 5.
A variety of condensing agents can be used for the coupling of a carrier to hapten. Glutaraldehyde, carbodiimide, maleimide activated ester, activated ester reagents containing thiol group or dithiopyridyl group, etc. are used for the coupling.
The condensation product is administered to warm-blooded animals either solely or together with carriers or diluents to the site in which the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.
The polyclonal antibody can be collected from the blood, ascites, etc., preferably from the blood of mammals immunized by the method described above.
The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.
The antisense polynucleotide comprising a complementary or substantially complementary nucleotide sequence, or a part of thereof, to the nucleotide sequence of a polynucleotide encoding the protein or partial peptide used in the present invention (which in the following description of the antisense polynucleotide, these DNAs are referred to sometimes as the DNA of the present invention) can be any antisense polynucleotide so long as it comprises a complementary or substantially complementary nucleotide sequence, or a part thereof, to that of the DNA of the present invention and capable of suppressing expression of the DNA. The antisense polynucleotide is preferably antisense DNA.
The nucleotide sequence substantially complementary to the DNA of the present invention includes, for example, a nucleotide sequence having at least about 70% homology, preferably at least about 80% homology, more preferably at least about 90% homology and most preferably at least about 95% homology, to the full-length nucleotide sequence or partial nucleotide sequence of the nucleotide sequence complementary to the DNA of the present invention (i.e., complementary strand to the DNA of the present invention). Particularly, (A) an antisense polynucleotide directed to translational inhibition is preferably an antisense polynucleotide having at least about 70% homology, preferably at least about 80% homology, more preferably at least about 90% homology and most preferably at least about 95% homology, to the complementary strand of the nucleotide sequence encoding the N-terminal site of the protein of the present invention (e.g., the nucleotide sequence around the initiation codon), in the entire nucleotide sequence of the complementary strand to the DNA of the present invention, and (B) an antisense polynucleotide directed to RNA decomposition by RNase H is preferably an antisense polynucleotide having at least about 70% homology, preferably at least about 80% homology, more preferably at least about 90% homology and most preferably at least about 95% homology, to the complementary strand of the entire nucleotide sequence of the intron-containing DNA of the present invention.
Specifically, the nucleotide sequence substantially complementary to the DNA of the present invention is an antisense polynucleotide having a complementary or substantially complementary nucleotide sequence, or a part thereof, to the nucleotide sequence of DNA comprising the nucleotide sequence represented by SEQ ID NO: 2, preferably an antisense polynucleotide having a complementary nucleotide sequence, or a part thereof, to the nucleotide sequence of DNA comprising the nucleotide sequence represented by SEQ ID NO: 2.
The antisense polynucleotide is composed of usually about 10 to 40 bases, preferably about 15 to 30 bases.
For preventing degradation by hydrolases such as nuclease etc., phosphoric acid residues (phosphates) of nucleotides constituting the antisense DNA may be substituted by chemically modified phosphoric acid residues such as phosphorothioate, methylphosphonate, phosphorodithionate etc. A sugar (deoxy ribose) of each nucleotide may be substituted by a chemically modified sugar structure such as a 2'-O-methylated structure, and a base moiety (pyrimidine, purine) of each nucleotide may have undergone chemical modification, and the antisense polynucleotide is not limited insofar as it hybridizes with DNA having the nucleotide sequence represented by SEQ ID NO: 2. These antisense polynucleotides can be produced by a known DNA synthesizer etc.

According to the present invention, antisense polynucleotides capable of inhibiting the replication or expression of the gene for the protein of the present invention can be designed and synthesized based on the nucleotide sequence information of the cloned or determined DNA encoding the protein. Such nucleotide (nucleic acid) is capable of hybridizing with RNA of the protein gene for the present invention to inhibit the synthesis or function of said RNA, or is capable of modulating or controlling the expression of the protein gene for the invention via interaction with the protein-associated RNA of the invention. Polynucleotides complementary to the selected sequences of the protein-associated RNA of the invention, and polynucleotides specifically hybridizable with the protein-associated RNA of the invention, are useful in modulating or controlling the expression of the protein gene for the invention in vivo and in vitro, and useful for the treatment or diagnosis of diseases. The term "corresponding" is used to mean homologous to or complementary to a particular sequence of the nucleotide, nucleotide sequence or nucleic acid including the gene. The term "corresponding" between nucleotides, nucleotide sequences or nucleic acids and proteins usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to, and hybridizable with, at least a part of the target region, can be denoted to be "antisense" to the polynucleotides in the target region. Examples of the antisense polynucleotides include polydeoxyribonucleotides containing 2-deoxy-D-ribose, polyribonucleotides containing D-ribose, any other type of polynucleotides which are N-glycosides of a purine or pyrimidine base, or other polymers having non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA: RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide of the present invention is RNA, DNA or a modified nucleic acid (RNA, DNA). Specific examples of the modified nucleic acid include, but are not limited to, sulfur and thiophosphate derivatives of nucleic acids and those resistant to degradation of polynucleoside amides or oligonucleoside amides. The antisense polynucleotides of the present invention can be modified preferably based on the following design, that is, by increasing the intracellular stability of the antisense polynucleotide, increasing the cell permeability of the antisense polynucleotide, increasing the affinity of the antisense polynucleotide for the targeted sense strand to a higher level, or minimizing the toxicity, if any, of the antisense polynucleotide.

Many of such modifications are known in the art, as disclosed in J. Kawakami et al., Pharm. Tech. Japan, Vol. 8, p. 247 or 395, 1992; Antisense Research and Applications, CRC Press, 1993; etc.

The antisense polynucleotide of the present invention may contain altered or modified sugars, bases or linkages, may also be provided in a specialized form such as liposomes or microspheres, may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the polynucleotide at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense polynucleotide can be examined using the transformant of the present invention, the gene expression system of the present invention in vivo and in vitro, or a translation system of the protein of the present invention in vivo and in vitro.

Hereinafter, the protein of the present invention, its partial peptides, or salts thereof (hereinafter sometimes referred to as the protein of the present invention), the polynucleotide encoding the protein of the present invention or its partial peptides (hereinafter sometimes referred to as the DNA of the present invention), the antibodies to the protein of the present invention, its partial peptides, or salts thereof (hereinafter sometimes referred to as the antibodies of the present invention) and the antisense polynucleotide of the DNA of the present invention (hereinafter sometimes referred to as the antisense polynucleotide of the present invention) are specifically described for the use or applications.

The protein of the present invention can be used as a diagnostic marker of atherosclerosis because its expression is increased at the legion of atherosclerosis. That is, the protein of the present invention (preferably, solubilized protein (i.e., the protein which is a solubilized protein of the protein, which is a single-pass transmenbrane protein, of the present invention) is useful as a marker for early diagnosis of atherosclerotic diseases or predictiing progression of atherosclerosis. Accordingly, a pharmaceutical comprising an antisense polynucleotide of a gene encoding the protein of the present invention, siRNA or shRNA of a gene encoding the protein of the present invention, compound or its salt inhibiting an activity of the protein of the present invention, compound or its salt inhibiting the expression of a gene encoding the protein of the present invention, compound or its salt inhibiting production of the protein of the present invention, or an antibody against to the protein of the present invention can be used as a prophylactic/therapeutic agent for atherosclerosis, a prophylactic/therapeutic agent for atherosclerotic diseases, etc.

Furthermore, the amount of GM3 synthesized by the protein of the present invention is increased in atherosclerotic diseases or at lesions of atherosclerosis, therefore, it can be used as a diagnostic marker of atherosclerosis. That is, it is useful as a marker for early diagnosis of atherosclerotic diseases or predictiing progression of atherosclerosis.

(1) Screening of a candidate compounds for diseases

The protein of the present invention is increased in its expression at lesions of atherosclerosis. Furthermore, inhibiting [hereinafter, "inhibiting the activity of the protein of the present invention", "inhibiting the expression of the gene encoding the protein of the present invention" and "inhibiting the production of the protein of the present invention" may be collectively referred to as "inhibiting the protein of the present invention". In addition, a compound or its salts itself that inhibits the protein of the present invention may be referred to as an inhibitor of the protein of the present invention. Furthermore, a prophylactic/therapeutic agent of the present invention includes compound or its salt itself that inhibits the protein of the present invention and a pharmaceutical composition comprising it.] the protein of the present invention causes improvement of atherosclerotic lesion. Accordingly, compound or its salt inhibiting the protein of the present invention can be used as, for example, prophylactic/therapeutic agent for atherosclerosis, a prophylactic/therapeutic agent for atherosclerotic diseases, etc. In addition, it can be thought to be applied for diabetes.

Accordingly, the protein of the present invention is useful as a reagent for screening a compound or its salt that inhibits the activity of the protein of the present invention. Also, the present invention provides a method of screening a compound or its salt that inhibits the activity of the protein of the present invention.

More specifically, for example, a method of screening a compound or its salt that inhibits the activity of the protein of the present invention, which comprises comparing (i) GM3 sythase activity of the cell having an ability to produce the protein of the present invention, cell extracts thereof or purified protein therefrom, with (ii) GM3 synthase activity of a mixture of the cell having an ability to produce the protein of the present invention, cell extracts thereof or purified protein therefrom and a test compound; a method of screening a compound or its salt that inhibits the activity of the protein of the present invention, which comprises comparing (iii) fat accumulating activity of the protein of the present invention, with (iv) fat accumulating activity of the mixture of the protein of the present invention and a test compound,etc., is used.

As the cells having an ability to produce the protein of the present invention, for example a host (transformant) transformed with a vector comprising the DNA encoding the protein of the present invention described above is used. As the host, animal cells such as COS7 cells, CHO cells, HEK293 cell are preferably used. In the screening, for example, a transformant expressing the protein of the present invention on a cell membrane or outside of the cell by culturing it is preferably used. The method for culturing the cells producible the protein of the present invention is the sameas the culturing method of transformant of the present invention described above.

Examples of the test compounds include peptides, proteins, antibodies, non-peptide compounds, synthetic compounds, fermentation products, cell extracts, plant extracts, animal tissue extracts, blood plasm, etc.

For example, a test compound which inhibits the activity in the above case (ii) by at least about 20%, preferably at least about 30%, more preferably at least about 50%, as compared with the activity in the above case (i) can be selected as a compound that inhibits the activity of the protein of the present invention.

The comopound inhibiting the activity of the protein of the present invention is useful as a pharmaceutical for inhibiting the physiological activity of the protein of the present invention.

The compounds or its salts, which are obtainable using the screening method or screening kit of the present invention, are compounds selected from, e.g., peptides, proteins, antibodies, non-peptide compounds, synthetic compounds, fermentation products, cell extracts, plant extracts, animal tissue extracts, blood plasma, and the like. As salts of these compounds, the same salts as those given above for the peptide of the present invention may be used.

Since expression of the gene encoding the protein of the present invention is increased in atherosclerosis, a compound or its salt that inhibits the expression of the gene encoding the protein of the present invention can be used as, for example, a prophylactic/therapeutic agent for atherosclerosis, a prophylactic/therapeutic agent for atherosclerotic diseases, etc.

Accordingly, the polynucleotide (for example, DNA) of the present invention is useful as a reagent for screening a compound or its salt that inhibits the expression of the gene encoding the protein of the present invention.

The screening method includes a screening method, which comprises comparing the case (v) where a cell having an ability to produce the protein of the present invention with the case (vi) where a cell having an ability to produce the protein of the present invention in the presence of a test compound.

In the above method, the expression level of the gene (specifically, the amount of the protein of the present invention or the amount of polynucleotide encoding the protein (e.g., mRNA)) is measured, and the expression level in the case (v) is compared with that in the case (vi).

The test compound and the cell having an ability to produce the protein of the present invention include the same specific examples as described above.

The amount of the protein can determined by measuring the protein present in a cell extract according to publicly known methods, for example, Western analysis, ELISA, or a modification of the known methods, by using the antibody recognizing the protein of the present invention.

The amount of the mRNA can determined by known methods, for example Northern hybridization using nucleic acid comprising SEQ ID NO: 2 or a part thereof as a probe, or PCR using nucleic acid comprising SEQ ID NO: 2 or a part thereof as a primer, or a modification of the known methods.

For example, a test compound which inhibits the expression level of the gene in the case (v) by at least about 20%, preferably at least about 30%, more preferably at least about 50%, as compared with the expression level in the case (vi), can be selected as a compound that inhibits the expression level of the gene encoding the protein of the present invention.

The screening kit of the present invention comprises the protein of the present invention, its partial peptide or a salt thereof, or a cell having an ability to produce the protein or partial peptide of the present invention.

The compounds or salts thereof, which are obtainable using the screening method or screening kit of the present invention, are the compounds or salts thereof selected from the test compounds described above, for example, peptides, proteins, antibodies, non-peptide compounds, synthetic compounds, fermentation products, cell extracts, plant extracts, animal tissue extracts, blood plasma, and the like, and compounds or salts thereof inhibiting the activity (for example, GM3 synthetic activity) of the protein of the present invention, or compounds or salts thereof inhibiting the expression of the gene encoding the protein of the present invention, compounds or salts thereof inhibiting the production of the protein of the present invention.

As salts of these compounds, the same salts as those given above for the protein of the present invention may be used.

Each the compound or its salt that inhibits the activity of the protein of the present invention, the compound or its salt that inhibits the expression of the gene encoding the protein of the present invention, and the compound or its salt that inhibits the production of the protein of the present invention is useful as a prophylactic/therapeutic agent for atherosclerosis, a prophylactic/therapeutic agent for atherosclerotic diseases, etc.

When the compound or its salt obtained by the screening method or screening kit of the present invention is used as the prophylactic/therapeutic agents supra, the compound or its salt can be formulated into a pharmaceutical composition in a conventional manner.

Examples of the composition for oral administration include solid or liquid preparations, specifically tablets (including sugarcoated tablets and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration that can be used are injections, suppositories, etc. and the injections include the form of intravenous, subcutaneous, intracutaneous, intramuscular, drip and intra-joint injections. Such injections are prepared by publicly known methods, e.g., by dissolving, suspending or emulsifying the aforesaid compound or its salts in a sterile aqueous or oily liquid medium used usually in injections. For the aqueous medium for injection, for example, physiological saline and isotonic solutions containing glucose and other adjuvant, etc. are used. Appropriate dissolution aids, for example, an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. may be used in combination. For the oily solution, for example, sesame oil, soybean oil, etc. are used, and dissolution aids such as benzyl benzoate, benzyl alcohol, etc. may be used in combination. The thus prepared liquid for injection is normally filled in an appropriate ampoule. The suppository used for rectal administration is prepared by mixing the aforesaid compound or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the compound described above is contained generally in a dose of approximately 5 to 500 mg per unit dosage form, approximately 5 to 100 mg especially for injections and approximately 10 to 250 mg for other preparations.

Each composition described above may further contain other active components unless formulation with the compound causes any adverse interaction. Since the thus obtained pharmaceutical preparation is safe and low toxic, and can be administered orally or parenterally to, for example, humans or warm-blooded animals (e.g., mouse, rat, rabbit, sheep, swine, bovine, horse, chicken, cat, dog, monkey, chimpanzee etc.).

The dose of the compound or its salt may vary depending on its action, target disease, subject of administration, route for administration, etc. When the compound of the present invention or its salt that inhibits the activity of the protein of the present invention is orally administered for example for the purpose of treatment of atheroscleotic disease (e.g., myocardial infarction, unstable angina pectoris, etc.), the compound or its salt is administered to adult (as 60 kg) generally in a daily dose of approximately 0.1 mg to 100 mg, preferably approximately 1.0 mg to 50 mg, more preferably approximately 1.0 to 20 mg. When the compound or its salt is parenterally administered, a single dose of the compound or its salt may vary depending on subject of administration, target disease, etc. When the compound or its salt that inhibits the activity of the protein of the present invention is administered in the form of an injection to adult (as 60 kg) for the purpose of treatment of atherosclerotic disease (e.g., myocardial infarction, unstable angina pectoris, etc.), it is advantageous to administer the compound or its salt by injection at atherosclerotic lesion generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

(2) Quantification of the protein of the present invention, its partial peptide, or its salt form

The antibodies of the present invention (hereinafter sometimes simply reffered to as the antibody of the present invention) are capable of specifically recognizing the protein of the present invention. Therefore, the antibodies can be used to quantify the protein of the present invention in a test fluid, especially for quantification by the sandwich immunoassay.

That is, the present invention provides, for example, the following quantification methods:

(i) A method of quantifying the protein of the present invention in a test fluid, which comprises competitively reacting the antibody of the present invention with the test fluid and a labeled form of the protein of the present invention, and measuring the ratio of the labeled form of the protein of the present invention bound to the antibody; and,
(ii) A method of quantifying the rprotein of the present invention in a test fluid, which comprises reacting the test fluid with the antibody of the present invention immobilized on a carrier and a labeled form of the antibody of the present invention simultaneously or sequentially, and measuring the activity of the labeling agent on the immobilized carrier.

In (ii) described above, it is preferred that one antibody recognizes the N-terminal region of the protein of the present invention, and another antibody reacts with the C-terminal region of the protein of the present invention.

Using monoclonal antibodies to the protein of the present invention (hereinafter sometimes referred to as the monoclonal antibodies of the present invention), the protein of the present invention can be assayed and also detected by tissue staining or the like. For this purpose, an antibody molecule itself may be used, or $F(ab')_2$, Fab' or Fab fractions of the antibody molecule may also be used.

Assay methods using antibodies to the protein of the present invention are not particularly limited. Any assay method can be used, so long as the amount of antibody, antigen, or antibody-antigen complex corresponding to the amount of antigen (e.g., the amount of the protein) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, for example, radioisotopes, enzymes, fluorescent substances, luminescent substances, etc. are employed. For the radioisotope, for example, [$^{125}$I], [$^{131}$I], [$^{3}$H] and [$^{14}$C] are used. As the enzyme described above, stable enzymes with high specific activity are preferred; for example, β-galactosidase, β-glucosidase, alkaline phosphatase, peroxidase, malate dehydrogenase and the like are used. For the fluorescent substance, fluorescamine and fluorescein isothiocyanate are used. For the luminescent substance, for example, luminol, luminol derivatives, luciferin, and lucigenin can be used. Furthermore, the biotin-avidin system may be used for binding antibody or antigen to the label. For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose, etc.; synthetic resin such as polystyrene, polyacrylamide, silicon, etc., and glass or the like are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity and so on.

In the methods of assaying the protein of the present invention by the sandwich method, antibodies that bind to different sites of the protein are preferably used as the monoclonal antibodies of the present invention for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc.

In the competitive method, antigen in a test fluid and labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the protein of the present invention or its salts are constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the protein of the present invention can be quantified with high sensitivity, using the antibodies of the present invention.

Furthermore, when an increase in the concentration of the protein of the present invention is detected by quantifying the concentration of the protein of the present invention using the antibody of the present invention, it can be diagnosed that there occur or will highly likely occur athrosclerotic diseases.

The antibodies of the present invention can also be used for specifically detecting the protein of the present invention present in test samples such as body fluids or tissues. The antibodies may also be used for preparation of antibody columns for purification of the protein of the present invention, for detection of the protein of the present invention in each fraction upon purification, and for analysis of the behavior of the protein of the present invention in the test cells.

(3) Gene diagnostic agent

By using the DNA of the present invention as a probe, an abnormality (gene abnormality) of the DNA or mRNA encoding the protein of the present invention or its partial peptide in human and other mammals (e.g., rats, mice, guinea pigs, rabbits, chickens, sheep, swine, bovine, horses, cats, dogs, monkeys, chimpanzees, etc.) can be detected. Therefore, the DNA of the present invention is useful as a gene diagnostic agent for diagnosing the damage of the DNA or mRNA, its mutation, or its decreased expression, or increased expression or overexpression of the DNA or mRNA.

The gene diagnosis described above using the DNA of the present invention can be performed by, for example, the publicly known Northern hybridization assay or PCR-SSCP assay (Genomics, 5, 874-879 (1989); Proceedings of the National Academy of Sciences of the United States of America, 86, 2766-2770 (1989)).

When overexpression of the protein is detected, e.g., by the Northern hybridization or when DNA mutation is detected by the PCR-SSCP assay, it can be diagnosed that it is highly likely to suffer from atherosclerotic diseases.

(4) Pharmaceutical comprising the antisense polynucleotide

The antisense polynucleotide of the present invention that binds complementarily to the DNA of the present invention to inhibit expression of the DNA is low-toxic and can suppress the functions of the protein of the present invention or the DNA of the present invention in the body, and can thus be used as a pharmaceutical.

When the antisense polynucleotide is used as the aforesaid pharmaceutical, it can be formulated into a pharmaceutical preparation and administered in publicly known methods.

For example, the antisense polynucleotide itself, or the antisense polynucleotide inserted into an appropriate vector such as retrovirus vector, adenovirus vector, adenovirus-associated virus vector, lentivirus vector, liposome derivative, etc., is administered orally or parenterally to human or other mammals (e.g., rat, rabbit, sheep, swine, bovine, cat, dog, monkey, etc.) in a conventional manner. The antisense polynucleotide may also be administered as it is, or prepared into pharmaceutical preparations together with physiologically acceptable carriers such as adjuvants to assist its uptake, and such preparations are administered by gene gun or through a catheter like a hydrogel catheter. Alternatively, the antisense polynucleotide may be prepared in the form of aerosol and intratracheally administered as an inhalant.

For the purposes of improvement of movement in the body, prolongation of the half-life, and improvement of incorporation into cells, the antisense polynucleotide may be administered such as intravenously or subcutaneously directly or after formulated into pharmaceutical preparations (injections) together with carriers such as liposomes. The dose of the antisense polynucleotide may vary depending on target disease, subject of administration, route for administration, etc. When the antisense polynucleotide of the present invention is administered for the purpose of treatment, the antisense polynucleotide is administered to adult (60 kg body weight) usually in a daily dose of approximately 0.1 to 100 mg.

In addition, the antisense polynucleotide may also be employed as an oligonucleotide probe for diagnosis to examine the presence of the DNA of the present invention in tissues or cells, or the states of its expression.

Double-stranded RNA (such as siRNA (small (short) interfering RNA), shRNA (small (short) hairpin RNA) against the polynucleotide of the present invetnion, etc.) comprising a part of RNA encoding the protein of the present invention or a ribozyme comprising a part of RNA encoding the protein of the present invention, etc., can suppress the expression of the gene of the present invention and can, in vivo, suppress the functions of the protein of the present invention or the DNA of the present invention, and can thus be used as a pharmaceutical.

According to known methods (for example, Nature, 411, 494, 2001), the double-stranded RNA can be produced by designing it on the basis of the sequence of the polynucleotide of the present invention.

According to known methods (for example, TRENDS in Molecular Medicine, 7, 221, 2001), the ribozyme can be produced by designing it on the basis of the sequence of the polynucleotide of the present invention. For example, the ribozyme can be produced by joining a known ribozyme sequence to a part of RNA encoding the protein of the present invention. The part of RNA encoding the protein of the present invention includes a sequence (RNA fragment) adjacent to the cleavage site on the RNA of the present invention which can be cleaved with a known ribozyme.

When the double-stranded RNA or the ribozyme is to be used as the aforesaid pharmaceutical, it can be formulated into a pharmaceutical preparation and administered in the same manner as for the antisense polynucleotide.

(5) Pharmaceutical comprising the antibody of the present invention

The antibody of the present invention can be used as a pharmaceutical. For this purpose, an antibody molecule itself may be used, or $F(ab')_2$, Fab' or Fab fractions of the antibody molecule may also be used.

Since the above described prophylactic/therapeutic agent for diseases is safe and low toxic, and can be administered orally or parenterally as liquid preparation as it is, or as a suitable form of pharmaceutical composition to humans or warm-blooded animals (e.g., rat, rabbit, sheep, swine, bovine, chicken, cat, dog, monkey, etc.). It is preferred to be administered as a vaccine according to the conventional manner.

The antibody of the present invention may be administered in itself or as an appropriate pharmaceutical composition. The pharmaceutical composition used for the administration described above can comprise the antibody and its salt and a pharmacologically acceptable carrier, a diluent or excipient. Such a composition is provided in the preparation suitable for oral or parenteral administration.

Examples of the composition for parenteral administration that can be used are injections, suppositories, vaccines etc. and the injections include the form of intravenous, subcutaneous, intracutaneous, intramuscular and drip injections. Such injections are prepared by publicly known methods. The injections can be prepared by dissolving, suspending or emulsifying the aforesaid antibody or its salts in a sterile aqueous or oily liquid medium used usually in injections. For the aqueous medium for injection, for example, physiological saline and isotonic solutions containing glucose and other adjuvant, etc. are used. Appropriate dissolution aids, for example, an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. may be used in combination. For the oily solution, for example, sesame oil, soybean oil, etc. are used, and dissolution aids such as benzyl benzoate, benzyl alcohol, etc. may be used in combination. The thus prepared liquid for injection is preferably filled in an appropriate ampoule. The suppository used for rectal administration may also be prepared by mixing the aforesaid antibody or its salts with conventional suppository base.

Examples of the composition for oral administration include solid or liquid preparations, specifically tablets (including sugarcoated tablet and film-coated tablets), pills, granules, powdery preparations, capsules (including soft capsules), syrup, emulsions, suspensions, etc. Such a composition is manufactured by publicly known methods and may comprises a vehicle, a diluent or an excipient conventionally used in the field of pharmaceutical preparations. Examples of the vehicle or excipient for tablets are lactose, starch, sucrose, magnesium stearate, etc.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is contained generally in a dose of approximately 5 to 500 mg per unit dosage form, approximately 5 to 100 mg especially for injections and approximately 10 to 250 mg for other preparations.

The dose of the antibody of the present invention may vary depending on subject of administration, target disease, symptom, route for administration, etc. When the antibody of the present invention is administered for the purpose of treatment or prevention in adult, it is advantageous to administer the antibody of the present invention by intravenous injection once to about 5 times per day, preferably once to 3 times per day, in a daily dose of approximately 0.01 to 20 mg/kg, preferably approximately 0.1 to 10 mg/kg, more preferably approximately 0.1 to 5 mg/kg. In other parenteral administration or oral administration, the corresponding dose can be administered. When the symptom is particularly severe, the dose may be increased depending on the symptom.

The antibody of the present invention is also useful as a diagnostic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), etc.

The antibody of the present invention may be administered in itself or as an appropriate pharmaceutical composition. The pharmaceutical composition used for the administration described above comprises the antibody or its salt and a pharmacologically acceptable carrier, a diluent or excipient. Such a composition is provided in the preparation suitable for oral or parenteral administration (e.g., intravascular injection, subcutaneous injection, etc.).

Each composition described above may further comprise other active components unless formulation with the antibody causes any adverse interaction.

(6) Regarding "a compound or its salts inhibiting the activity of the protein of the present invention"; "a compound or its salts inhibiting the expression of the gene encoding the protein of the present invention" and "a compound or its salts inhibiting the production of the protein of the present invention"

"A compound or its salts inhibiting the activity of the protein of the present invention" and "a compound or its salts inhibiting the production of the protein of the present invention" are used as, for example, a prophylactic/therapeutic agent for atherosclerosis, a prophylactic/therapeutic agent for atherosclerotic diseases, etc.

"A compound or its salts inhibiting the expression of the gene encoding the protein of the present invention" are used as, for example, a prophylactic/therapeutic agent for atherosclerosis, a prophylactic/therapeutic agent for atherosclerotic diseases, etc. Besides, a prophylactic/therapeutic agent for atherosclerosis, a prophylactic/therapeutic agent for atherosclerotic diseases in the present application comprises "a compound or its salts inhibiting the activity of the protein of the present invention" itself; "a compound or its salts inhibiting the expression of the gene encoding the protein of the present invention" itself; "a compound or its salts inhibiting the production of the protein of the present invention" itself; and a pharmaceutical composition comprising any of these compounds.

The prophylactic/therapeutic agent can be manufactured as described above.

(7) DNA transferred animal

The present invention provides a non-human mammal bearing the DNA encoding the protein of the present invention, which is exogenous (hereinafter simply referred to as the exogenous DNA of the present invention) or its mutant DNA (sometimes simply referred to as the exogenous mutant DNA of the present invention).

Thus, the present invention provides:

(1) a non-human mammal having the exogenous DNA of the present invention or its mutant DNA;
(2) the mammal according to (1), wherein the non-human mammal is a rodent;
(3) the mammal according to (2), wherein the rodent is a mouse or rat; and
(4) a recombinant vector comprising the exogenous DNA of the present invention or its mutant DNA and capable of expression in a mammal.

The non-human mammal having the exogenous DNA of the present invention or its mutant DNA (hereinafter simply referred to as the DNA transferred animal of the present invention) can be created by transferring the desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase) by standard means such as the calcium phosphate method, the electric pulse method, the lipofection method, the agglutination method, the microinjection method, the particle gun method, the DEAE-dextran method, etc. Also, it is possible to transfer the exogenous DNA of the present invention into a somatic cell, a living organ, a tissue cell or the like, by the DNA transfer methods, and utilize it for cell culture, tissue culture, etc. In addition, these cells may be fused with the above-described germinal cell by a publicly known cell fusion method to create the DNA transferred animal of the present invention.

Examples of the non-human mammal that can be used include bovine, swine, sheep, goat, rabbits, dogs, cats, guinea pigs, hamsters, mice, rats and the like. Above all, preferred are rodents, especially mice (e.g., C57BL/6 strain, DBA2 strain, etc. for a pure line, and B6C3F$_1$ strain, BDF$_1$ strain, B6D2F$_1$ strain, BALB/c strain, ICR strain, etc.) or rats (Wistar, SD, etc.) and the like, since they are relatively short in ontogeny and life cycle from a standpoint of creating model disease animals, and are easy in breeding. "Mammals" relating to a recombinant vector that can be expressed in mammals include human etc. in addition to the aforesaid non-human mammals.

The exogenous DNA of the present invention refers not to the DNA of the present invention inherently possessed by the non-human mammals, but to the DNA of the present invention that is once isolated and extracted from mammals.

The mutant DNA of the present invention includes mutants resulting from variation (e.g., mutation, etc.) in the nucleotide sequence of the original DNA of the present invention, specifically DNA resulting from base addition, deletion, substitution with other bases, etc. and further including abnormal DNA.

The abnormal DNA is intended to mean the DNA that expresses abnormal protein of the present invention. For example, such a DNA that expresses the protein suppressing the functions of the normal protein of the present invention, or the like is used.

The exogenous DNA of the present invention may be any one of those derived from a mammal of the same species as, or a different species from, the mammal as the target animal. In transfecting the DNA of the present invention to the target animal, it is generally advantageous to use the DNA as a DNA construct in which the DNA is ligated downstream from a promoter capable of expressing the DNA in the target animal. For example, in the case of transfecting the human DNA of the present invention, a DNA transferred mammal that expresses the DNA of the present invention to a high level can be prepared by microinjecting a DNA construct (e.g., vector, etc.), which comprises the human DNA of the present invention ligated downstream of the various promoters, which is derived from various mammals (e.g., rabbits, dogs, cats, guinea pigs, hamsters, rats, mice, etc.) having the DNA of the present invention highly homologous to the human DNA, capable of expressing the DNA, into a fertilized egg of the target mammal, e.g., into a fertilized egg of mouse, .

As expression vectors for the protein of the present invention, Escherichia coli-derived plasmids, Bacillus subtilis-derived plasmids, yeast-derived plasmids, bacteriophages such as 1 phage, etc., retroviruses such as Moloney leukemia virus, etc., animal viruses such as vaccinia virus, baculovirus, etc. are used. Of these vectors, Escherichia coli-derived plasmids, Bacillus subtilis-derived plasmids, yeast-derived plasmids, etc. are preferably used.

Examples of these promoters for regulating expression of the DNA described above include (1) promoters for DNA derived from viruses (e.g., simian virus, cytomegalovirus, Moloney leukemia virus, JC virus, breast cancer virus, poliovirus, etc.), and (2) promoters derived from various mammals (humans, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na, K-ATPase), neurofilament light chain, metallothioneins I and IIA, metalloproteinase 1 tissue inhibitor, MHC class I antigen (H-2L), H-ras, renin, dopamine β-hydroxylase, thyroid peroxidase (TPO), polypeptide chain elongation factor 1α (EF-1α), b actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Preferable among these are cytomegalovirus promoters, human peptide elongation factor 1α (EF-1α) promoters, human and chicken β actin promoters etc., which can achieve high expression in the whole body.

It is preferred that the vectors described above have a sequence for terminating the transcription of the desired messenger RNA in the DNA transferred animal (generally called a terminator), for example, a sequence of each DNA derived from viruses and various mammals. SV40 terminator of the simian virus, etc. are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The normal translational region of the protein of the present invention can be prepared as the whole or a part of genomic DNA from DNA derived from liver, kidney, thyroid cells, fibroblasts etc. derived from humans or mammals (for example, rabbit, dog, cat, guinea pig, hamster, rat, mouse etc.) and a wide variety of commercial DNA libraries, or from complementary DNA prepared by a known method from RNA derived from liver, kidney, thyroid cells, fibroblasts etc. as a starting material. As the extraneous abnormal DNA, a translational region can be prepared by point mutation of the normal translational region of the protein obtained from the above cells or tissues.

The translational region can be prepared, as a DNA construct capable of being expressed in the transgenic animal, by a conventional DNA engineering technique, in which the DNA is ligated downstream from the aforesaid promoter and if desired, upstream from the translation termination site.

The exogenous DNA of the present invention is transferred at the fertilized egg cell stage in a manner such that the DNA is certainly present in all the germinal cells and somatic cells of the target mammal. The fact that the exogenous DNA of the present invention is present in the germinal cells of the animal prepared by DNA transfer means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal, in which the normal exogenous DNA of the present invention has been transferred, can be passaged as the DNA-bearing animal under ordinary rearing environment, by confirming that the exogenous DNA is stably retained by mating.

By the transfection of the exogenous DNA of the present invention at the fertilized egg cell stage, the DNA is retained to be excess in all of the germinal and somatic cells of the target mammal. The fact that the exogenous DNA of the present invention is excessively present in the germinal cells of the prepared animal after transfection means that all of the offspring of the animal prepared have the exogenous DNA of the present invention excessively in all of the germinal cells and somatic cells thereof. The offspring of the animal of this kind that inherits the exogenous DNA of the present invention excessively have the DNA of the present invention in all of the germinal cells and somatic cells thereof.

By obtaining a homozygotic animal having the transferred DNA in both of homologous chromosomes and mating a male and female of the animal, all offspring can be passaged to excessively retain the DNA.

In a non-human mammal bearing the normal DNA of the present invention, the normal DNA of the present invention is expressed to a high level, and may eventually develop the hyperfunction of the protein of the present invention by promoting the functions of endogenous normal DNA. Therefore, the animal can be utilized as a pathologic model animal for such a disease. Specifically, using the normal DNA transferred animal of the present invention, it becomes possible to elucidate the hyperfunction of the protein of the present invention and to clarify the pathological mechanism of the disease associated with the protein of the present invention and to examine how to treat these diseases.

Furthermore, since a mammal transferred with the exogenous normal DNA of the present invention exhibits an increasing symptom of the free protein of the present invention, the animal is usable for screening of prophylactic/therapeutic agents for the disease associated with the protein of the present invention, for example, prophylactic/therapeutic agent for atherosclerotic diseases.

On the other hand, non-human mammal having the exogenous abnormal DNA of the present invention can be passaged under normal breeding conditions as the DNA-bearing animal by confirming the stable retaining of the exogenous DNA via crossing. In addition, the objective exogenous DNA can be utilized as a starting material by inserting the objective exogenous DNA into the plasmid described above. The DNA construct with a promoter can be prepared using conventional DNA engineering techniques. The transfer of the abnormal DNA of the present invention at the fertilized egg cell stage is preserved to be present in all of the germinal and somatic cells of the mammals to be targeted. The fact that the abnormal DNA of the present invention is present in the germinal cells of the animal after DNA transfer means that all of the offspring of the prepared animal have the abnormal DNA of the present invention in all of the germinal and somatic cells. The offspring of such an animal that inherits the exogenous DNA of the present invention has the abnormal DNA of the present invention in all the germinal and somatic cells. A homozygous animal having the introduced DNA on both of homologous chromosomes can be acquired and then by mating these male and female animals, all the offspring can be bred to have the DNA.

Since the non-human mammal having the abnormal DNA of the present invention expresses the abnormal DNA of the present invention at a high level, the animal may cause the function inactive type inadaptability of the protein of the present invention by inhibiting the functions of the endogenous normal DNA, and can be utilized as its disease model animal. For example, using the abnormal DNA-transferred animal of the present invention, it is possible to elucidate the mechanism of the function inactive type inadaptability of the protein of the present invention and to study a method for treatment of this disease.

In its specific applicability, the transgenic animal of the present invention expressing the abnormal DNA of the present invention to a high level is also expected to serve as a model for the elucidation of the mechanism of the functional inhibition (dominant negative effect) of the normal protein of the present invention by the abnormal protein of the present invention in the function inactive type inadaptability of the protein of the present invention.

A mammal bearing the abnormal exogenous DNA of the present invention has a symptom of increasing the protein of the present invention in a free form, and is thus utilizable in a test of screening prophylactic/therapeutic agents for the function inactive type inadaptability of the protein of the present invention.

Other potential applications of two kinds of the DNA transferred animals of the present invention described above further include:

(1) Use as a cell source for tissue culture,
(2) Elucidation of the relation to a peptide that is specifically expressed or activated by the protein of the present invention, by direct analysis of DNA or RNA in tissues of the DNA transferred animal of the present invention or by analysis of the peptide tissues expressed by the DNA,
(3) Research on the function of cells derived from tissues that are usually cultured only with difficulty, using cells in tissues bearing the DNA cultured by a standard tissue culture technique,
(4) Screening a drug that enhances the functions of cells using the cells described in (3) above, and
(5) Isolation and purification of the mutant protein of the present invention and preparation of an antibody thereto.

Furthermore, clinical conditions of a disease associated with the protein of the present invention, including the function inactive type inadaptability to the protein of the present invention, can be examined by using the DNA transferred animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transferred cell by withdrawing each organ from the DNA transferred animal of the present invention, mincing the organ and degrading it with a proteinase such as trypsin, etc., followed by establishing the line of culturing or cultured cells. Furthermore, the DNA transferred animal of the present invention can be used to identify cells capable of producing the protein of the present invention, and to study in association with apoptosis, differentiation or proliferation or on the mechanism of signal transduction in these properties to inspect any abnormality therein. Thus, the DNA transferred animal can provide an effective research material for investigation of the protein of the present invention and for investigation of the function and effect thereof.

To develop a drug for the treatment of diseases associated with the protein of the present invention, including the function inactive type inadaptability to the protein of the present invention, using the DNA transferred animal of the present invention, an effective and rapid method for screening a drug for the treatment of the diseases can be provided by using the method for inspection, the method for quantification etc. described above. It is also possible to investigate and develop a method for DNA therapy for the treatment of diseases associated with the protein of the present invention, using the DNA transferred animal of the present invention or a vector capable of expressing the exogenous DNA of the present invention.

(8) Knockout animals

The present invention provides a non-human mammalian embryonic stem cell, wherein the DNA of the present invention is inactivated, and a non-human mammal, which DNA is barely expressed.

That is, the present invention provides:

(1) A non-human embryonic stem cell in which the DNA of the present invention is inactivated;
(2) The embryonic stem cell according to (1), wherein the DNA is inactivated by introducing a reporter gene (e.g., β-galactosidase gene derived from Escherichia coli);
(3) The embryonic stem cell according to (1), which is resistant to neomycin;
(4) The embryonic stem cell according to (1), wherein the non-human mammal is a rodent;
(5) An embryonic stem cell according to (4), wherein the rodent is mouse;
(6) A non-human mammal deficient in expressing the DNA of the present invention, wherein the DNA of the present invention is inactivated;
(7) The non-human mammal according to (6), wherein the DNA is inactivated by inserting a reporter gene (e.g., β-galactosidase derived from Escherichia coli) therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method for screening a compound or its salt that promotes or inhibits the promoter activity for the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell (hereinafter merely referred to as ES cell) that suppresses the ability of the non-human mammal to express the DNA by artificially mutating the DNA of the present invention, or the DNA has no substantial ability to express the polypeptide of the present invention (hereinafter sometimes referred to as the knockout DNA of the present invention) by substantially inactivating the activities of the polypeptide of the present invention encoded by the DNA.

As the non-human mammal, the same examples as described above are used. Methods for artificially mutating the DNA of the present invention include, for example, deletion of a part or all of the DNA sequence and insertion of or substitution with other DNA, by genetic engineering. By these mutations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating a DNA strand (hereinafter simply referred to as targeting vector) having a DNA sequence, which terminates gene transcription (e.g., polyA additional signal, etc.), inserted in the intron between exons to inhibit the synthesis of complete messenger RNA and eventually destroy the gene to a chromosome of the subject animal by, e.g., homologous recombination. The knockout ES cell of the present invention can be obtained by selecting the thus-obtained ES cells by the Southern hybridization analysis with a DNA sequence on or near the DNA of the present invention as a probe, or to PCR analysis with a DNA sequence on the targeting vector and another DNA sequence near the DNA of the present invention which is not included in the targeting vector as primers.

The parent ES cells to inactivate the DNA of the present invention by homologous recombination, etc. may be of a strain already established as described above, or may be originally established in accordance with a modification of the publicly known method by Evans and Kaufman. For example, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum availability in the C57BL/6 mouse has been improved by crossing with DBA/2, may be preferably usedto obtain an alternative pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is desirable to identify sexes as soon as possible also in order to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, whereas a karyotype analysis requires about $10^6$ cells conventionally; therefore, the first selection of ES cells at the early stage of culture can be based on sex identification, and male cells can be selected early, which saves a significant amount of time at the early stage of culture.

Second selection can be achieved by, for example, number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.

Although the embryonic stem cell line thus obtained usually shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1-10000 U/ml) on appropriate feeder cells such as STO fibroblasts, treated with a trypsin/EDTA solution (normally about 0.001 to about 0.5% trypsin/about 0.1 to about 5 mM EDTA, preferably about 0.1 % trypsin/1 mM EDTA) at the time of passage to obtain separate single cells, which are then seeded on freshly prepared feeder cells. This passage is normally conducted every 1 to 3 days; it is desirable that cells be observed at passage and cells found to be morphologically abnormal in culture, if any, be abandoned.

By allowing ES cells to reach a high density in mono-layers or to form cell aggregates in suspension under appropriate conditions, it is possible to spontaneously differentiate them to various cell types, for example, pariental muscle, visceral muscles, cardiac muscle or the like (M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985). The cells deficient in expression of the DNA of the present invention, which are obtainable from the differentiated ES cells of the present invention, are useful for cell biological study of the functions of the protein of the present invention.

The non-human mammal deficient in expression of the DNA of the present invention can be identified from a normal animal by measuring the amount of mRNA in the subject animal by a publicly known method, and indirectly comparing the degrees of expression.

As the non-human mammal, the same examples described above are used. With respect to the non-human mammal deficient in expression of the DNA of the present invention, the DNA of the present invention can be made knockout by transferring a targeting vector, prepared as described above, to mouse embryonic stem cells or mouse oocytes, and conducting homologous recombination in which a targeting vector DNA sequence, wherein the DNA of the present invention is inactivated by the transfer, is replaced with the DNA of the present invention on a chromosome of a mouse embryonic stem cell or mouse oocyte.

The knockout cells with the DNA of the present invention disrupted can be identified by Southern hybridization analysis with a DNA sequence on or near the DNA of the present invention as a probe, or by PCR analysis using a DNA sequence on the targeting vector and another DNA sequence derived from mouse, which is not included in the targeting vector, as primers. When non-human mammalian embryonic stem cells are used, a cell line wherein the DNA of the present invention is inactivated by homologous recombination is cloned; the resulting cloned cell line is injected to, e.g., a non-human mammalian embryo or blastocyte, at an appropriate stage such as the 8-cell stage. The resulting chimeric embryos are transplanted to the uterus of the pseudopregnant non-human mammal. The prepared animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between the heterozygotes.

When an oocyte is used, a DNA solution may be injected, e.g., to the nucleus of the oocyte by microinjection thereby to obtain a transgenic non-human mammal having a targeting vector introduced in a chromosome thereof. From such transgenic non-human mammals, those having a mutation at the locus of the DNA of the present invention can be obtained by selection based on homologous recombination.

As described above, individuals in which the DNA of the present invention is rendered knockout permit passage rearing under ordinary rearing conditions, after the individuals obtained by their crossing have proven to have been knockout the DNA.

Furthermore, the genital system may be obtained and maintained by conventional methods. That is, by crossing male and female animals each having the inactivated DNA, homozygous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell in which the DNA of the present invention is inactivated is very useful for preparing a non-human mammal deficient in expression of the DNA of the present invention.

Since the non-human mammal in which the DNA of the present invention is inactivated lacks various biological activities derived from the protein of the present invention, such an animal can be a disease model suspected of inactivated biological activities of the protein of the present invention and thus, offers an effective study to investigate causes for and therapy for these diseases.

(8a) Methods for screening of compounds having therapeutic and/or prophylactic effects for diseases caused by deficiency, damages, etc. of the DNA of the present invention

The non-human mammal deficient in expression of the DNA of the present invention can be employed for the screening of compounds having therapeutic and/or prophylactic effects for diseases caused by deficiency, damages, etc. of the DNA of the present invention.

That is, the present invention provides a method for screening of a compound having therapeutic and/or prophylactic effects for diseases caused by deficiency, damages, etc. of the DNA of the present invention, which comprises administering a test compound to the non-human mammal deficient in expression of the DNA of the present invention and observing and measuring a change occurred in the animal.

As the non-human mammal deficient in expression of the DNA of the present invention, which can be employed for the screening method, the same examples as given hereinabove are given.

Examples of the test compounds include peptides, proteins, antibodies, non-peptide compounds, synthetic compounds, fermentation products, cell extracts, vegetable extracts, animal tissue extracts, blood plasma and the like and these compounds may be novel compounds or publicly known compounds.

Specifically, the non-human mammal deficient in the expression of the DNA of the present invention is treated with a test compound, and by comparison with an intact animal for control, a change in each organ, tissue, disease conditions, etc. of the animal is used as an index to assess the therapeutic and/or prophylactic effects of the test compound. For treating an animal to be tested with a test compound, for example, oral administration, intravenous injection, etc. are applied and the treatment is appropriately selected depending upon conditions of the test animal, properties of the test compound, etc. Furthermore, an amount of a test compound administered can be selected depending on administration route, nature of the test compound, and the like.

For example, in the case of screening a compound having a therapeutic and/or prophylactic effect for atherosclerotic diseases, a test compound is administered to the non-human mammal deficient in expression of the DNA of the present invention. Then, onset level of the atherosclerotic diseases or curing level of the atherosclerotic diseases of the organ described above is measured with passage of time.

In the screening method, when a test compound is administered to an animal to be tested and found to reduce the blood sugar level of the animal to at least about 10%, preferably at least about 30% and more preferably at least about 50%, the test compound can be selected as a compound having the therapeutic and/or prophylactic effect for the above-mentioned diseases.

The compound obtained using the above screening method is a compound selected from the test compounds described above and exhibits a therapeutic and/or prophylactic effect for the diseases caused by deficiencies, damages, etc. of the protein of the present invention. Therefore, the compound can be employed as a safe and low toxic pharmaceutical for the treatment and prevention of these diseases. Furthermore, compounds derived from such a compound obtained by the above screening can be likewise employed. Diseases caused by overexpression of the protein of the present invention include, for example, atherosclerotic diseases. Accordingly, a compound or its salts inhibiting the activity of the protein of the present invention, a compound or its salts inhibiting the expression of the gene encoding the protein of the present invention, a compound or its salts inhibiting the production of the protein of the present invention, etc. can be used as a prophylactic/therapeutic agent for atherosclerotic diseases.

The compound obtained by the screening method may be used in the form of salts with physiologically acceptable acids (e.g., inorganic acids or organic acids) or bases (e.g., alkali metal), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical containing the compound obtained by the above screening method or salts thereof may be manufactured in a manner similar to the method for preparing the pharmaceutical comprising the protein of the present invention described hereinabove.

Since the pharmaceutical thus obtained is safe and low toxic, it can be administered to human or other mammals (e.g., rat, mouse, guinea pig, rabbit, sheep, swine, bovine, horse, cat, dog, monkey, etc.).

Although the amount of the compound or its salt to be administered varies depending upon particular disease, subject to be administered, route of administration, etc., but when the compound is orally administered, the compound is generally administered to an adult patient with atherosclerotic diseases (as 60 kg body weight) in a dose of about 0.1 mg/day to about 100 mg/day, preferably about 1.0 mg/day to about 50 mg/day, more preferably about 1.0 mg to about 20 mg. For parenteral administration, a single dose of the compound varies depending upon subject to be administered, target disease, etc., but when the compound is administered to an adult patient with atherosclerotic diseases (as 60 kg body weight) in the form of an injection, it is generally advantageous to administer the compound intravenously in a dose of about 0.01 mg/day to about 30 mg/day, preferably about 0.1 mg/day to about 20 mg/day, more preferably about 0.1 mg/day to about 10 mg/day. As for other animals, the compound can be administered in the above amount with converting it into that for the body weight of 60 kg.

(8b) Method for screening a compound that promotes or inhibits the activities of a promoter to the DNA of the present invention

The present invention provides a method for screening a compound or its salt that promotes or inhibits the activities of a promoter to the DNA of the present invention, which comprises administering a test compound to a non-human mammal deficient in expression of the DNA of the present invention and detecting expression of the reporter gene.

In the screening method described above, as the non-human mammal deficient in expression of the DNA of the present invention, an animal in which the DNA of the present invention is inactivated by introducing a reporter gene and the reporter gene can be expressed under control of a promoter to the DNA of the present invention is used from the aforementioned non-human mammal deficient in expression of the DNA of the present invention.

The same examples of the test compound apply to specific compounds used for the screening.

As the reporter gene, the same specific examples described above apply, and β-galactosidase (lacZ), soluble alkaline phosphatase gene, luciferase gene and the like are preferably employed.

Since a reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal), which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After that, by washing the tissue preparation with 1 mM EDTA/PBS solution, the β-galactosidase reaction is terminated, and the color formed is observed. Alternatively, mRNA encoding lacZ may be detected in a conventional manner.

The compound or salts thereof obtained using the above screening method, are compounds that are selected from the test compounds described above and that promote or inhibit the promoter activity to the DNA of the present invention.

The compound obtained by the screening method above may be used in the form of salts with physiologically acceptable acids (e.g., inorganic acids or organic acids) or bases (e.g., alkali metal), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Since the compounds or salts thereof that enhance the promoter activity to the DNA of the present invention can promote the expression of the protein of the present invention, or can promote the functions of the protein, they are useful as safe and low toxic pharmaceuticals for the treatment and/or prevention of atherosclerotic diseases, etc. In addition, compound derived from the compounds obtained by the screening above may be likewise employed.

A pharmaceutical containing the compounds or salts thereof obtained by the screening method may be manufactured in a manner similar to the method for preparing the pharmaceutical containing the protein of the present invention described above.

Since the pharmaceutical preparation thus obtained is safe and low toxic, it can be administered to human or other mammals (e.g., rat, mouse, guinea pig, rabbit, sheep, swine, bovine, horse, cat, dog, monkey, etc.).

The dose of the compound or salts thereof varies depending on target disease, subject to be administered, route for administration, etc.; for example, when the compound that regulates (enhances or inhibits; preferably inhibits) the promoter activity to the DNA of the present invention is orally administered, the dose is normally about 0.1 to about 100 mg, preferably about 1.0 to about 50 mg, more preferably about 1.0 to about 20 mg per day for adult patient with atherosclerotic diseases (as 60 kg body weight). In parenteral administration, a single dose of the compound varies depending on subject to be administered, target disease, etc. but when the compound that regulates (enhances or inhibits; preferably inhibits) the promoter activity to the DNA of the present invention is administered in the form of injection, it is advantageous to administer the compound intravenously at a single dose of about 0.01 to about 30 mg/day, preferably about 0.1 to about 20 mg/day, more preferably about 0.1 to about 10 mg/day for adult patient with atherosclerotic diseases (as 60 kg body weight). For other animal species, the corresponding dose as converted per 60 kg weight can be administered.

As stated above, the non-human mammal deficient in expression of the DNA of the present invention is extremely useful for screening the compound or its salt that promotes or inhibits the activity of a promoter to the DNA of the present invention and can greatly contribute to the elucidation of causes for various diseases suspected of deficiency in expression of the DNA of the present invention and for the development of medicament for prevention and/or treatment of these diseases.

Furthermore, a so-called transgenic animal (gene introduced animal) can be prepared by using DNA containing a promoter region of the protein of the present invention, ligating genes encoding various proteins downstream and injecting the same into oocyte of an animal. It is then possible to synthesize the protein therein specifically and study its activity in vivo. When an appropriate reporter gene is ligated to the promoter site above and a cell line that express the gene is established, the resulting system can be utilized as the survey system for a low molecular compound having the action of specifically promoting or inhibiting the in vivo productivity of the protein per se of the present invention.

(9) Pharmaceutical comprising the protein of the present invention or the DNA of the present invention

Regarding the protein of the present invention, where the DNA encoding the protein of the present invention is abnormal or deficient, or where the expression level of the protein of the present invention is reduced, there occur a variety of diseases caused by these factors.

Accordingly, the protein of the present invention and the DNA of the present invention can be used as safe pharmaceuticals such as prophylactic/therapeutic agents for diseases described above.

For example, when there is a patient having reduced amount of the protein of the present invention or lacking of the protein of the present invention in the living body, (a) DNA encoding the protein of the invention is administered into the patient to express the protein of the invention in the living body, (b) the DNA is inserted into target cells to express the protein of the invention and the cells are transplanted to the patient, or (c) the protein of the invention is administered into the patient, whereby the role of the protein of the invention can be exhibited sufficiently or normally in the patient.

When the DNA of the present invention is used as the prophylactic/therapeutic agents described above, the DNA itself is administered; alternatively, the DNA is inserted into an appropriate vector such as retrovirus vector, adenovirus vector, adenovirus-associated virus vector, etc. and then administered into humans or warm-blooded animals in a conventional manner. The DNA of the present invention may also be administered as naked DNA, or in the form of a preparation prepared with physiologically acceptable carriers such as adjuvants to assist its uptake by gene gun or through a catheter such as a catheter with a hydrogel.

When the protein of the present invention is used as the aforesaid prophylactic/therapeutic agent, the protein is advantageously used on a purified level of at least 90%, preferably at least 95%, more preferably at least 98% and most preferably at least 99%.

For example, the protein of the present invention can be used orally in the form of tablets which may be sugar coated if necessary and desired, capsules, elixirs, microcapsules etc., or parenterally in the form of injectable preparations such as a sterile solution in water or with other pharmaceutically acceptable liquid, or a suspension. These preparations can be manufactured for example by mixing the protein of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose or saccharin, and a flavoring agent such as peppermint, akamono oil or cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredient in a vehicle such as water for injection, with a naturally occurring vegetable oil such as sesame oil, coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate solubilizer such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a solubilizer such as benzyl benzoate, benzyl alcohol, etc. It may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The vector in which the DNA of the present invention is inserted may also be prepared into pharmaceutical preparations in a manner similar to the procedures above, and such preparations are generally used parenterally.

Since the thus obtained pharmaceutical preparation is safe and low toxic, and can be administered to, for example, warm-blooded animals (e.g., human, rat, mouse, guinea pig, rabbit, chicken, sheep, swine, bovine, horse, cat, dog, monkey, chimpanzee etc.). The dose of such as the protein of the present invention may vary depending on target disease, subject of administration, route for administration, etc. When the protein of the present invention is orally administered, the protein is administered to adult (as 60 kg) generally in a daily dose of approximately 0.1 mg to 100 mg, preferably approximately 1.0 mg to 50 mg, more preferably approximately 1.0 to 20 mg. When the protein is parenterally administered, a single dose of the protein may vary depending on subject of administration, target disease, etc. When the protein is administered in the form of an injection to adult (as 60 kg), it is advantageous to inject the protein at the affected lesion generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| I | : inosine |
| R | : adenine (A) or guanine (G) |
| Y | : thymine (T) or cytosine (C) |
| M | : adenine (A) or cytosine (C) |
| K | : guanine (G) or thymine (T) |
| S | : guanine (G) or cytosine (C) |
| W | : adenine (A) or thymine (T) |
| B | : guanine (G), guanine (G) or thymine (T) |
| D | : adenine (A), guanine (G) or thymine (T) |
| V | : adenine (A), guanine (G) or cytosine (C) |
| N | : adenine (A), guanine (G), cytosine (C) or thymine (T), or unknown or other bases |
| RNA | : ribonucleic acid |
| mRNA | : messenger ribonucleic acid |
| dATP | : deoxyadenosine triphosphate |
| dTTP | : deoxythymidine triphosphate |
| dGTP | : deoxyguanosine triphosphate |
| dCTP | : deoxycytidine triphosphate |
| ATP | : adenosine triphosphate |
| EDTA | : ethylenediaminetetraacetic acid |

SDS : sodium dodecyl sulfate
BHA : benzhydrilamine
PMBHA : p-methylbenzhydrilamine
DCM : dichloromethane
TFA : trifluoroacetic acid
DIEA : diisopropylethylamine
Gly or G : glycine
Ala or A : alanine
Val or V : valine
Leu or L : leucine

Ile or I : isoleucine
Ser or S : serine
Thr or T : threonine
Cys or C : cysteine
Met or M : methionine
Glu or E : glutamic acid
Asp or D : aspartic acid
Lys or K : lysine
Arg or R : arginine
His or H : histidine
Phe or F : phenylalanine
Tyr or Y : tyrosine
Trp or W : tryptophan
Pro or P : proline
Asn or N : asparagine
Gln or Q : glutamine
pGlu : pyroglutamic acid
Tyr(I) : 3-iode tyrosine
* : corresponding stop codon
Me : methyl
Et : ethyl
Bu : butyl
Ph : phenyl
TC : thiazolidine-4(R)-carboxamide

The substituents, protective groups and reagents, which are frequently used throughout the specification, are shown by the following abbreviations.

Tos : p-toluenesulfonyl
CHO : formyl
Bzl : benzyl
$Cl_2Bl$ : 2,6-dichlorobenzyl
Bom : benzyloxymethyl
Z : benzyloxycarbonyl
Cl-Z : 2-chlorobenzyloxycarbonyl
Br-Z : 2-bromobenzyloxycarbonyl
Boc : t-butoxycarbonyl
DNP : dinitrophenol
Trt : trityl
Bum : t-butoxymethyl
Fmoc : N-9-fluorenylmethoxycarbonyl
HOBt : 1-hydroxybenztriazole
HOOBt : 3,4-dihydro-3-hydroxy-4-oxo-1,2,3-benzotriazine
HONB : 1-hydroxy-5-norbornene-2,3-dicarboximide
DCC : N,N'-dicyclohexylcarbodiimide
DMP : N,N'-dimethylformamide
Pbf : 2,2,4,6,7-pentamethyldihydrobenzofuran-5-sulfonyl

Clf : 2-chlorotrityl
But : t-buthyl
Met(O) : methionine sulfoxide

The sequence identification numbers in the sequence listing of the specification indicates the following sequence, respectively.

[SEQ ID NO: 1]
This shows the amino acid sequence of GM3 synthase.
[SEQ ID NO: 2]
This shows the base sequence of the DNA encoding the GM3 synthase having the amino acid sequence represented by SEQ ID NO: 1. This sequence corresponds to the $278^{th}$ to $1363^{rd}$ base sequence of the base sequence described in GenBank: NM_003896.
[SEQ ID NO: 3]
This shows the base sequence of the sense primer used in Example 1.
[SEQ ID NO: 4]
This shows the base sequence of the antisense primer used in Example 1.
[SEQ ID NO: 5]
This shows the base sequence of shRNA used in Example 5.
[SEQ ID NO: 6]
This shows the base sequence of shRNA used in Example 5.

## EXAMPLES

The present invention is described in detail below with reference to EXAMPLES, but is not deemed to limit the scope of the present invention thereto.

## EXAMPLE 1

Study on expression of GM3 synthase at atherosclerotic lesion in rabbit aorta

Five-week aged New Zealand white rabbit (Kbl:NZW) was bred with RC-4 diet supplemented with 0.5% cholesterol for 8 (eight) weeks, and was further bred with RC-4 feed for 4 (four) weeks. Under anesthesia, after venisection from carotid artery, sampling from the aortic arch to the thoracic aorta was carried out. In cold physiological saline, adipose tissue, connective tissue and the like were trimmed from blood vessel. The blood vessel was stored in cold RNAlater (QIAGEN). Aorta was divided into two parts, one including many atherosclerotic lesions and other including little lesions by visual check. Using RNeasy Fibrous Tissue Midi Kit (QIAGEN), total RNA was prepared. Using TaqMan Reverse Transcription Reagents (Applied Biosystems), cDNA was prepared. By carrying out PCR reaction with TaqMan Universal PCR Master Mix (Applied Biosystems) and agarose gel electrophoresis, expression levels were semi-quantitatively compared. PCR primers were designed from the information on the nucleotide sequence of a partial sequence of rabbit GM3 synthase. Using TAATCGGAAGTGGCGGAATAC (SEQ ID NO: 3) as a sense primer and CTCGGCCCCAGAACCTTGACC (SEQ ID NO: 4) as a antisense primer, the PCR reaction was performed under conditions of annealing temperature at 55°C and 34 cycles. From the results of agarose gel electrophoresis, when the comparison was performed in the same individual, it was demonstrated that the mRNA expression of GM3 synthase was obviously increased in the aorta having a lot of lesions compared to that having little lesions. Therefore, it was suggested that that GM3 synthase activity is enhanced at the atherosclerotic lesion.

## EXAMPLE 2

Effects of interferon γ on the expression of mouse GM3 synthase in mouse peritoneal macrophage cells

In accordance with the method by Hakamada et al. [Jikken-igaku (Experimental Medicine), Supplementary Volume, Vol. 14, No. 12, "Junnkan Kenkyu Protocol (Protocol for study on circulation)", pp. 49-52, 1996], from C57BL/6j mouse after the intraperitoneal injection of thioglycolate, peritoneal macrophage was prepared. The cells were suspended in 10% FBS-DMEM culture medium to be 1.5-2 x $10^6$ cells/mL, and were seeded on 12-well plate at 1 mL/well. After overnight culture at 37°C in the presence of 5% $CO_2$, the cells were washed with PBS. The 1% FBS-DMEM culture medium containing mouse recombinant IFN-γ at prescribed concentration was added to the cells, and cultivation was performed for 24 hours.

After washing the cells once with PBS, using RNeasy Mini Kit (QIAGEN) total RNA was prepared. Subsequently, using TaqMan Reverse Transcription Reagents (Applied Biosystems) cDNA was prepared. By TaqMan PCR method Using TaqMan Universal PCR Master Mix (Applied Biosystems), the expression level of GM3 synthase was quantified. As TaqMan probe and primers for mouse GM3 synthase, Assays-on-Demand (Mm00488232_ml) supplied from Applied Biosystems was used.

In quantification, an expression level of GAPDH was used as internal standard. By ΔΔCt method, relative value was calculated based on the conditions without treatment of IFN-γ as 1 (one). The result are shown in TABLE 1.

(TABLE 1)

| mIFN-γ (U/mL) | Expression |
|---|---|
| 0 | 1 |
| 1 | 4 |
| 10 | 25 |

It was indicated that an expression level of mouse GM3 synthase was dose-dependently increased by treatment of IFN-γ known as the atherogenic cytokine. From this result, a possibility that increased expression of GM3 synthase at atherosclerotic lesion is caused by IFN-γ was suggested.

## EXAMPLE 3

Effects of interferon γ on the expression of human GM3 synthase in the THP-1 cells

THP-1 cells suspended in RPMI-1640 culture medium containing phorbol 12-myristeate 13-acetate (final concentration: 100 mM), in which 10% FBS, 25 mM HEPES and Penicillin/Streptomycin were comprised, were seeded on 24-well plate at 1.0 x $10^6$ cells/well and treated, for 3 (three) days to differentiate into macrophage-like cells. After washing the cells with PBS, RPMI-1640 culture medium containing human recombinant IFN-γ at a prescribed concentration was added to the cells. Subsequently, the cells were cultured for 24 hours.

After washing the cells once with PBS, by the similar method to that of EXAMPLE 2, an expression level of human GM3 synthase was quantified. As TaqMan probe and primers for human GM3 synthase, Assays-on-Demand (Hs00187405_ml) supplied from Applied Biosystems was used.

In quantification, an expression level of GAPDH was used as internal standard. By ΔΔCt method, relative value was calculated based on the conditions without treatment of IFN-γ as 1 (one). The result are shown in TABLE 2.

(TABLE 2)

| hIFN-γ (ng/mL) | Expression level |
|---|---|
| 0 | 1 |
| 3 | 1.9 |
| 10 | 2.6 |
| 30 | 2.4 |

From this result, it was found that the expression level of human GM3 synthase was increased dependent on the concentration of interferon γ treated.

## EXAMPLE 4

1) A method of screening GM3 synthase inhibitors (1)

The cell line stably expressing human GM3 synthase, which was established by transducing human GM3 synthase gene into RAW264.7 strain purchased from ATCC (Raschke WC , et al., Cell, Vol. 15, pp. 261-267, 1978) with retrovirus vector, was cultured in 10% FBS- DMEM culture medium. After culturing to confluent state, the cells were washed once with PBS and harvested. After weighing wet cells, the cells were suspended in 3 (three) volumes of buffer solution (15 mM sodium cacodilate (pH6.5), 5% glycerol). By ultrasonication, the cells were disrupted, and the supernatant, of which cell debris was excluded by centrifugation, was used as a crude enzyme solution. Protein concentration of the crude enzyme solution was determined using BCA protein assay kit(BIO RAD, Inc.).

BODIPY-lactosylceramide (Molecular Probes, Inc.) in chloroform solution was dried in Eppendorf tube under nitrogen flow. 4 x reaction buffer solution consisting of 400 mM sodium cacodylate (pH6.5), 1.6% Triton X-100, 40 mM $MgCl_2$ and 8 mM 2,3 dehydro-2-deoxy-N-acetylneuraminic acid was added to the tube. After vortexing, it was sufficiently dissolved by ultrasonication for 20 seconds in sonicator bath and was used as a substrate solution.

Ten μl of substrate solution, 10 μl of 800 μM CMP-sialic acid, 10 μl of crude enzyme solution and 10 μl of sample were admixed and followed by incubating at 37°C for one hour. As a sample, cytidine mono phosphate (CMP), wherein the inhibitory effect to GM3 synthase activity is reported, was used (Biochem. Biophys. Res. Commun., Vol. 193, pp. 585-590, 1993). One milliliter of chloroform/methanol mixture (2:1) was added to the reaction mixture to terminate the reaction. After mixing, ultrasonication was done for 30 minutes in sonicator bath. Supernatant was recovered by centrifugation at 15,000 rpm for 5 minutes. Pellet was further washed with 200 μl of chloroform/methanol mixture (2:1) and recovered its supernatant by centrifugation at 15,000 rpm for 5 minutes. Collected supernatant was dried under nitrogen flow, and residue was re-suspended in 50 μl of chloroform/methanol mixture (2:1). Twenty μl of the supernatant isolated by centrifugation was spotted on HPTLC (Merck, Inc.) and was developed with solvent system consisting of chloroform/methanol/0.2% $CaCl_2$ aqueous solution (50:45:10). After development, fluorescent intensity was measured with LAS-1000 (Fuji Film, Inc.). Fluorescent intensity of both the substrate, BODIPY-lactosylceramide and the product, BODIPY-GM3 was measured, respectively to calculate a ratio of GM3 produced to lactosylceramide added. The activity was calculated as an amount of GM3 (nmol) produced/hour/mg of protein in the crude enzyme solution.

$$\text{GM3 synthase activity (nmol/mg/hr)}$$
$$= \text{BODIPY-GM3 (AU)/(BODIPY-lactosylceramide (AU) + BODIPY-GM3 (AU))}$$
$$\text{x 10 nmol x 1/(protein weight (mg) x reaction time (hr))}$$

AU means a numerical value determined with LAS-1000.

The results of measurement are shown in TABLE 3.

(TABLE 3)

| CMP (mM) | GM3 synthase activity (nmol/mg/hr) |
|---|---|
| 0 | 73 |
| 0.1 | 46 |
| 0.3 | 27 |
| 1 | 12 |
| 3 | 5 |
| 10 | 7 |

Using the assay system described above, GM3 synthase activity and its inhibition by CMP were determined. By adding test compounds instead of CMP, compounds that inhibit GM3 synthase can be screened.

2) A method of screening GM3 synthase inhibitors (2)

Coating of lactosylceramide, the substrate for GM3 synthase to Phospholipid FlashPlate (PerkinElmer, Inc.) was carried out by the method as described below. 200 μM lactosylceramide-reaction buffer solution consisting of 100 mM sodium cacodylate (pH6.5) and 10 mM $MgCl_2$ was dispensed on the plate at 200 μl/well and followed by standing at room temperature for overnight. After washing the plate once with PBS, 30 μM lactosylceramide-reaction buffer solution was dispensed at 70 μl/well.

Ten microliter of the crude enzyme solution prepared by the procedure in EXAMPLE 4, 10 μl of CMP solution and 10 μl of 500 μM CMP-[$^{14}$C] sialic acid (Amersham; 5 x $10^4$ cpm/well) were admixed and incubated at 37°C for 3 (three) hours. Radioactivity was measured by Topcount (PerkinElmer, Inc.).

A well, in which buffer solution for cell suspension was added instead of the crude enzyme was used as a blank. A value obtained by subtract the cpm value of blank well from the cpm value of sample well was used as a value for enzyme activity.

Relative value was calculated based on the conditions without treatment of CMP as 1 (one).

The results are shown in TABLE 4.

(TABLE 4)

| CMP (mM) | GM3 synthase activity (relative value) |
|---|---|
| 0 | 1.00 |
| 0.04 | 1.01 |
| 0.12 | 0.73 |
| 0.4 | 0.58 |
| 1.2 | 0.32 |
| 4 | 0.28 |

By the assay system described above, GM3 synthase activity and inhibition with CMP were measured. By adding test compounds instead of CMP, compounds that inhibit GM3 synthase can be screened.

EXAMPLE 5

Effects of the shRNA expressing retrovirus on GM3 synthase activity in RAW264.7 strain

RAW264.7 strain purchased from ATCC (Raschke WC , et al., Cell, Vol. 15, pp. 261-267, 1978) was suspended in 10% FBS-DMEM culture medium to be 1 x $10^5$ cells/ml, and was seeded on 12-well plate at 1 ml/well. After overnight incubation at 37°C in the presence of 5% $CO_2$, retrovirus, which expressed sequences for shRNA (short hairpin RNA), SH1 (SEQ ID NO: 5) or SH3 (SEQ ID NO: 6), was infected to RAW264.7 strain. After overnight culture, the medium was replaced with 10%FBS-DMEM culture medium and the cells were cultured to confluent state. Using these cells, in accordance with the method in EXAMPLE 4, crude enzyme was prepared to assay GM3 synthase activity. Based on the value of the enzyme activity from RAW264.7 strain as 1 (one), the value from the virus-infected cells was calculated as relative value. The results are shown in TABLE 5.

(TABLE 5)

| infected retrovirus | GM3 synthase activity (relative value) |
|---|---|
| none | 1.0 |
| SH1 | 0.3 |
| SH3 | 0.5 |

As a result, it was confirmed that in RAW264.7 strain infected with shRNA expressing retrovirus, GM3 synthase activity was reduced.

EXAMPLE 6

Effects of knock-down for GM3 synthase on cholesterol efflux from RAW264.7 strain

RAW264.7 strain infected with the retrovirus that expresses shRNA targeting GM3 synthase was suspended in 10% FBS-DMEM culture medium to be 1.5-2 x $10^6$ cells/ml and was seeded on 12-well plate at 1 mL/well. After overnight culture at 37°C in the presence of 5% $CO_2$, the cells were washed with PBS. Then, after adding 1% FBS-DMEM culture medium, which contained [$^3$H] cholesterol-labeled βVLDL (final concentration: 150 μg of total cholesterol/ml), the cells were cultured for overnight. The cells were washed twice with 0.2% BSA-PBS and further washed with PBS. After replacing the medium with 0.1% BSA- DMEM culture medium, the cells were cultivated for one day. After replacing 20 μg/ml of Apolipoprotein A1 containing 0.1% BSA-DMEM culture medium, the cells were cultured for 6 hours. Cultured supernatant was collected, and radioactivity of the supernatant, from which cell debris was removed by centrifugation, was measured with the liquid scintillation counter. To the cells, of which supernatant was discarded, 0.1 N NaOH solution which contains 0.1% SDS was added. After incubation at 37°C for overnight, radioactivity of the cell lysate was measured with the liquid scintillation counter. A ratio of the radioactivity of the culture supernatant to the summation of the radioactivity of the culture supernatant and the cell lysate was calculated. The ratio was designated a cholesterol efflux activity. Based on the value from RAW264.7 strain as 1 (one), the value from the virus-infected cells was calculated as relative value. The results are shown in TABLE 6.

(TABLE 6)

| infected retrovirus | cholesterol transporting activity (relative value) |
|---|---|
| none | 1.0 |
| SH1 | 1.3 |
| SH3 | 1.1 |

As a result, it became clear that cholesterol efflux from RAW264.7 strain infected with shRNA expressing retrovirus, in which GM3 synthase activity was reduced, was increased. From the fact, it is indicated that an compound for inhibiting GM3 synthase activity or its expression enhances a cholesterol transport and shows anti-atherosclerotic effect.

INDUSTRIAL APPLICABILITY

GM3 synthase used in the present invention, of which expression is specifically increased in atherosclerotic lesion, is a diagnostic marker for atherosclerosis. Therefore, a compound that inhibits a GM3 synthase activity, or a salt thereof, a compound that inhibits an expression of GM3 synthase gene, or a salt thereof, a compound that inhibits a production of GM3 synthase, or a salt thereof, and others can be used safely as an prophylactic/therapeutic agent for, for example, atherosclerotic and/or atherosclerotic diseases such as cerebral arteriopathy (e.g., brain infarction, brain hemorrhage); coronary artery diseases (e.g., ischemic heart disease such as cardiac infarction, angina cordis); aortic diseases such as aortic aneurysm, aortic dissection; renal arterial diseases such as nephrosclerosis, renal failure caused by nephrosclerosis; peripheral arterial diseases such as atherosclerosis obliterans, and the like.

## Claims

**1.** A medicament for prevention/treatment of atherosclerosis comprising a GM3 synthase inhibitor.

**2.** A medicament for prevention/treatment of atherosclerosis comprising an inhibitor for expression of GM3 synthase gene.

**3.** The medicament of claim 1 or 2, wherein GM3 synthase is a protein comprising the same or substantially the same amino acid sequence as that of SEQ ID NO: 1, its partial peptide or a salt thereof.

**4.** The medicament of claim 1 or 2, wherein GM3 synthase is a protein consisting of the amino acid sequence of SEQ ID NO: 1, or a salt thereof.

**5.** An antisense polynucleotide comprising a base sequence complement to or substantially complement to that of the polynucleotide coding for the protein having the same or substantially the same amino acid sequence as that of SEQ ID NO: 1, or a partial peptide thereof.

**6.** A medicament comprising the antisense polynucleotide of claim 5.

**7.** The medicament of claim 6, which is a medicament for prevention/treatment of atherosclerosis.

**8.** An siRNA or shRNA to the polynucleotide coding for the protein having the same or substantially the same amino acid sequence as that of SEQ ID NO: 1, or a partial peptide thereof.

**9.** A medicament comprising the siRNA or shRNA of claim 8.

**10.** The medicament of claim 9, which is a medicament for prevention/treatment of atherosclerosis.

**11.** A medicament for prevention/treatment of atherosclerosis comprising an antibody to GM3 synthase.

**12.** A diagnostic product for atherosclerosis comprising an antibody to GM3 synthase.

**13.** A diagnostic product for atherosclerosis comprising a polynucleotide coding for the protein having the same or substantially the same amino acid sequence as that of SEQ ID NO: 1, or a partial peptide thereof.

**14.** A method of diagnosing atherosclerosis which is **characterized by** using an antibody to GM3 synthase or a polynucleotide coding for the protein having the same or substantially the same amino acid sequence as that of SEQ ID NO: 1, or a partial peptide thereof.

**15.** Use of antibody to GM3 synthase or a polynucleotide coding for the protein having the same or substantially the same amino acid sequence as that of SEQ ID NO: 1, or a partial peptide thereof, for manufacturing a diagnostic product for atherosclerosis.

**16.** A method of diagnosing atherosclerosis which is **characterized by** quantifying GM3 in blood plasma of mammal.

**17.** Use of GM3 for diagnostic marker of atherosclerosis.

**18.** A method of diagnosing atherosclerosis which is **characterized by** quantifying GM3 synthase in blood plasma of mammal.

**19.** Use of GM3 synthase for diagnostic marker of atherosclerosis.

**20.** A method of screening a prophylactic/therapeutic agent for atherosclerosis which is **characterized by** using the protein having the same or substantially the same amino acid sequence as that of SEQ ID NO: 1, its partial peptide or a salt thereof.

**21.** A kit for screening a prophylactic/therapeutic agent for atherosclerosis which is **characterized by** comprising the protein having the same or substantially the same amino acid sequence as that of SEQ ID NO: 1, its partial peptide or a salt thereof.

**22.** A method of screening a prophylactic/therapeutic agent for atherosclerosis which is **characterized by** using a polynucleotide coding for the protein having the same or substantially the same amino acid sequence as that of SEQ ID NO: 1, or a partial peptide thereof.

**23.** A kit for screening a prophylactic/therapeutic agent for atherosclerosis which is **characterized by** comprising a polynucleotide coding for the protein having the same or substantially the same amino acid sequence as that of SEQ ID NO: 1, or a partial peptide thereof.

**24.** A method of screening a prophylactic/therapeutic agent for atherosclerosis which is **characterized by** assaying an activity of the protein comprising the same or substantially the same amino acid sequence as that of SEQ ID NO: 1, its partial peptide or a salt thereof.

**25.** A method of screening a prophylactic/therapeutic agent for atherosclerosis which is **characterized by** quantifying the protein comprising the same or substantially the same amino acid sequence as that of SEQ ID NO: 1, its partial peptide or a salt thereof.

**26.** A method of quantifying the protein comprising the same or substantially the same amino acid sequence as that of SEQ ID NO: 1, its partial peptide or a salt thereof, which is **characterized by** using an antibody to GM3 synthase.

**27.** A method of screening a prophylactic/therapeutic agent for atherosclerosis which is **characterized by** quantifying a polynucleotide coding for the protein having the same or substantially the same amino acid sequence as that of SEQ ID NO: 1, or a partial peptide thereof.

**28.** A method for preventing/treating atherosclerosis which is **characterized by** inhibiting GM3 synthase.

**29.** A method for preventing/treating atherosclerosis which is **characterized by** inhibiting an expression of GM3 synthase.

**30.** A method for preventing/treating atherosclerosis which is **characterized by** administering an effective amount of GM3 synthase inhibitor to mammals.

**31.** A method for preventing/treating atherosclerosis which is **characterized by** administering an effective amount of inhibitor for expression of GM3 synthase to mammals.

**32.** Use of GM3 synthase inhibitor for manufacturing a prophylactic/therapeutic agent for atherosclerosis.

**33.** Use of inhibitor for expression of GM3 synthase for manufacturing a prophylactic/therapeutic agent for atherosclerosis.

**INTERNATIONAL SEARCH REPORT**

International application No.
PCT/JP2005/000733

A. CLASSIFICATION OF SUBJECT MATTER
Int.Cl[7] A61K45/00, 48/00, 31/7088, 39/395, A61P9/10, 43/00, C12N15/11, C12Q1/48, 1/68, G01N33/15, 33/50, 33/53

According to International Patent Classification (IPC) or to both national classification and IPC

B. FIELDS SEARCHED

Minimum documentation searched (classification system followed by classification symbols)
Int.Cl[7] A61K45/00, 48/00, 31/7088, 39/395, A61P9/10, 43/00, C12N15/11, C12Q1/48, 1/68, G01N33/15, 33/50, 33/53

Documentation searched other than minimum documentation to the extent that such documents are included in the fields searched

Electronic data base consulted during the international search (name of data base and, where practicable, search terms used)
CAPLUS(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| A | JP 2000-333682 A (Seikagaku Corp.), 05 December, 2000 (05.12.00), & US 2003/0087396 A1 | 1-13,15, 20-25,27,32, 33 |
| A | JP 11-018778 A (Seikagaku Corp.), 26 January, 1999 (26.01.99), & EP 890645 A2 | 1-13,15, 20-25,27,32, 33 |
| A | FUKUMOTO, Satoshi et al., Expression cloning of mouse cDNA of CMP-NeuAc: lactosylceramide .alpha.2,3-sialyltransferase, an enzyme that initiates the synthesis of gangliosides, Journal of Biological Chemistry, 1999, 274(14), 9271 to 9276 | 1-13,15, 20-25,27,32, 33 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13 April, 2005 (13.04.05) | Date of mailing of the international search report<br>10 May, 2005 (10.05.05), |
| Name and mailing address of the ISA/<br>Japanese Patent Office<br>Facsimile No. | Authorized officer<br><br>Telephone No. |

Form PCT/ISA/210 (second sheet) (January 2004)

**INTERNATIONAL SEARCH REPORT**

International application No.
PCT/JP2005/000733

C (Continuation). DOCUMENTS CONSIDERED TO BE RELEVANT

| Category* | Citation of document, with indication, where appropriate, of the relevant passages | Relevant to claim No. |
|---|---|---|
| A | Yasutake USUKINE, "Toshitsu GB3 no Kino Kaimei ni Mukete -Seigosei Sogaizai no Katsuyo-", Nippon Yuka Gakkaishi, 1997, 46(10), 1075 to 1086 | 1-13,15, 20-25,27,32, 33 |
| A | MELKERSON-WATSON, Lyla J. et al., Purification to apparent homogeneity by immunoaffinity chromatography and partial characterization of the GM3 ganglioside-forming enzyme, CMP-sialic acid:lactosylceramide .alpha.2, 3-sialyltransferase (SAT-1), from rat liver Golgi, Journal of Biological Chemistry, 1991, 266(7), 4448-57 | 1-13,15, 20-25,27,32, 33 |

Form PCT/ISA/210 (continuation of second sheet) (January 2004)

**INTERNATIONAL SEARCH REPORT**

International application No.
PCT/JP2005/000733

**Box No. II Observations where certain claims were found unsearchable (Continuation of item 2 of first sheet)**

This international search report has not been established in respect of certain claims under Article 17(2)(a) for the following reasons:

1. [X] Claims Nos.: 14, 16-19, 28-31
because they relate to subject matter not required to be searched by this Authority, namely:
Claims 14, 16 to 19 and 28 to 31 pertain to methods for treatment of the human body by surgery or therapy and diagnostic methods and thus relate to a subject matter which this International Searching Authority is not required, under the provisions of Article 17(2)(a)(i) (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
The inventions according to claims 1 to 13, 15, 20 to 25, 27, 32 and 33 relate to a drug for preventing or treating arteriosclerosis which contains a GM3 synthase inhibitor or an agent suppressing the expression of a GM3 synthase gene and a diagnostic for arteriosclerosis which contains an antibody against GM3 synthase. On the other hand, the invention according to claim 26 relates to a method of quantifying a protein having an amino acid sequence which is the same or substantially the same as the amino acid sequence represented by SEQ ID NO:1, its partial peptide or its salt characterized by using an antibody against GM3 synthase. However, it cannot be recognized that the above-described method of quantifying a protein, (continued to extra sheet)

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.:
Claims 1 to 13, 15, 20 to 25, 27, 32 and 33.

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

INTERNATIONAL SEARCH REPORT

International application No.
PCT/JP2005/000733

Continuation of Box No.II-1 of continuation of first sheet(2)

of the PCT and Rule 39.1(iv) of the Regulations under the PCT, to search.

Continuation of Box No.III of continuation of first sheet(2)

its partial peptide or its salt characterized by using an antibody against GM3 synthase is a method specifically applied to the production of a drug for preventing or treating arteriosclerosis which contains a GM3 synthase inhibitor or an agent suppressing the expression of a GM3 synthase gene or a diagnostic for arteriosclerosis which contains an antibody against GM3 synthase. Thus, there is no common matter seemingly being a special technical feature in the meaning within the second sentence of PCT Rule 13.2. Therefore, no technical relevancy can be found out between these invention groups differing from each other in the meaning within PCT Rule 13. Such being the case, it does not appear that there is a technical relationship between these groups of inventions involving one or more of the same or corresponding special technical features and, therefore, these groups of inventions are not considered as relating to a group of inventions so linked as to form a single general inventive concept.

<Subject of search>

Claims 1, 2, 32 and 33 relate to a drug for preventing or treating arteriosclerosis which contains, as the active ingredient, a compound defined by a desired property, i.e., "a GM3 synthase inhibitor" or "an agent suppressing the expression of a GM3 synthase gene". Although claims 1, 2, 32 and 33 involve any compounds having these properties, it is recognized that only small parts of the claimed compounds are supported by the description in the meaning within PCT Article 6 and disclosed therein in the meaning within PCT Article 5.

Although the common technical knowledge at the point of the application is taken into consideration, the scope of the compounds having the property as "a GM3 synthase inhibitor" or "an agent suppressing the expression of a GM3 synthase gene" cannot be specified. Thus, claims 1, 2, 32 and 33 do not comply with the requirement for clearness in the meaning within PCT Article 6 too.

Such being the case, the search was made on the relationship between "inhibition of GM3 synthase" and "inhibition of the expression of a GM3 synthase gene" and "arteriosclerosis".

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0378624 A


### Non-patent literature cited in the description


- **FUSTER, V. et al.** *N.Engl. J. Med.,* 1992, vol. 326, 242-250
- **EHARA, S. et al.** *Circulation,* 2001, vol. 103, 1955-1960
- *Arterioscler Thromb Vasc Biol.,* 1995, vol. 15, 1607-1615
- **HARA, A. ; TAKETOMI, T.** *J. Biochem.,* 1991, vol. 109, 904-908
- **BOBRYSHEV, Y.V. et al.** *Biochim. Biophys. Acta,* 2001, vol. 1535, 87-99
- **PROKAZOVA N et al.** *Biochem Biophys. Res. Commun.,* 1991, vol. 177, 582-587
- **KOLTER, T. et al.** *J. Biol. Chem.,* 2002, vol. 277, 25859-25862
- **TETTAMANTI, G. et al.** *Biochimie,* 2003, vol. 85, 423-437
- **TAGAMI, S. et al.** *J. Biol. Chem.,* 2002, vol. 277, 3085-3092
- **ISHII, A. et al.** *J. Biol. Chem.,* 1998, vol. 273, 31652-31655
- **YAMASHITA, T. et al.** *Proc. Natl. Acad. Sci. USA,* 2003, vol. 100, 3445-3449
- *Methods in Enzymology,* 2000, vol. 311, 82-94
- **WANG, X.Q. et al.** *J. Biol. Chem.,* 2002, vol. 277, 47028-47034
- **INOKUCHI, J. et al.** *J. Cell Phys.,* 1989, vol. 141, 573-583
- **M. BODANSZKY ; M.A. ONDETTI.** Peptide Synthesis. Interscience Publishers, 1966
- **SCHROEDER ; LUEBKE.** The Peptide. Academic Press, 1965
- **NOBUO IZUMIYA et al.** Peptide Gosei-no-Kiso to Jikken (Basics and experiments of peptide synthesis. Maruzen Co,
- Tanpakushitsu no Kagaku (Chemistry of Proteins. **HARUAKI YAJIMA ; SHUNPEI SAKAKIBARA.** Seikagaku Jikken Koza (Biochemical Experiment. 1977, vol. IV, 205
- Zoku Iyakuhin no Kaihatsu (A sequel to Development of Pharmaceuticals. Hirokawa Shoten, vol. 14
- **J. SAMBROOK et al.** Molecular Cloning. Cold Spring Harbor Lab. Press, 1989
- Molecular Cloning. Cold Spring Harbor Lab. Press, 1989
- *Proc. Natl. Acad. Sci. U.S.A.,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **VAUGHN, J. L. et al.** *In Vivo,* 1977, vol. 13, 213-217
- **MAEDA et al.** *Nature,* 1985, vol. 315, 592
- *Proc. Natl. Acad. Sci. U.S.A.,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proc. Natl. Acad. Sci. U.S.A.,* 1978, vol. 75, 1929
- *Bio/Technology,* 1988, vol. 6, 47-55
- Shin Saibo Kogaku Jikken Protocol (New Cell Engineering Experimental Protocol. Shujunsha, 1995, 263-267
- *Virology,* 1973, vol. 52, 456
- Molecular Genetics. Cold Spring Harbor Laboratory, 1972, 431-433
- *Nature,* 1975, vol. 256, 495
- **J. KAWAKAMI et al.** *Pharm. Tech. Japan,* 1992, vol. 8, 247 or 395
- Antisense Research and Applications. CRC Press, 1993
- Radioimmunoassay. Kodansha, 1974
- Sequel to the Radioimmunoassay. Kodansha, 1979
- Enzyme immonoassay. Igakushoin, 1978
- Immunoenzyme assay. Igakushoin, 1982
- Immunoenzyme assay. Igakushoin, 1987
- *Methods in ENZYMOLOGY,* vol. 70
- *METHODS IN ENZYMOLOGY,* vol. 73

- *METHODS IN ENZYMOLOGY,* vol. 74
- *METHODS IN ENZYMOLOGY,* vol. 84
- *METHODS IN ENZYMOLOGY,* vol. 92
- *METHODS IN ENZYMOLOGY,* vol. 121
- *Genomics,* 1989, vol. 5, 874-879
- *Proceedings of the National Academy of Sciences of the United States of America,* 1989, vol. 86, 2766-2770
- *Nature,* 2001, vol. 411, 494
- *TRENDS in Molecular Medicine,* 2001, vol. 7, 221
- **M. J. EVANS ; M. H. KAUFMAN.** *Nature,* 1981, vol. 292, 154
- **G. R. MARTIN.** *Proc. Natl. Acad. Sci. U.S.A.,* 1981, vol. 78, 7634
- **T. C. DOETSCHMAN et al.** *Journal of Embryology Experimental Morphology,* 1985, vol. 87, 27
- **HAKAMADA et al.** Junnkan Kenkyu Protocol (Protocol for study on circulation. *Jikken-igaku (Experimental Medicine,* 1996, vol. 14 (12), 49-52
- **RASCHKE WC et al.** *Cell,* 1978, vol. 15, 261-267
- *Biochem. Biophys. Res. Commun.,* 1993, vol. 193, 585-590

```
SEQUENCE LISTING

<110> Takeda Pharmaceutical Company Limited

<120> DRUG FOR PREVENTING AND TREATING ARTERIOSCLEROSIS

<130> G06-0038

<150> PCT/JP2005/000733
<151> 2005-01-14

<150> JP 2004-9383
<151> 2004-01-16

<160> 6

<210> 1
<211> 362
<212> PRT
<213> Homo sapience

<400> 1
Met Arg Arg Pro Ser Leu Leu Leu Lys Asp Ile Leu Lys Cys Thr Leu
                5                   10                  15
Leu Val Phe Gly Val Trp Ile Leu Tyr Ile Leu Lys Leu Asn Tyr Thr
            20                  25                  30
Thr Glu Glu Cys Asp Met Lys Lys Met His Tyr Val Asp Pro Asp His
        35                  40                  45
Val Lys Arg Ala Gln Lys Tyr Ala Gln Gln Val Leu Gln Lys Glu Cys
    50                  55                  60
Arg Pro Lys Phe Ala Lys Thr Ser Met Ala Leu Leu Phe Glu His Arg
65                  70                  75                  80
Tyr Ser Val Asp Leu Leu Pro Phe Val Gln Lys Ala Pro Lys Asp Ser
                85                  90                  95
Glu Ala Glu Ser Lys Tyr Asp Pro Pro Phe Gly Phe Arg Lys Phe Ser
            100                 105                 110
Ser Lys Val Gln Thr Leu Leu Glu Leu Leu Pro Glu His Asp Leu Pro
        115                 120                 125
Glu His Leu Lys Ala Lys Thr Cys Arg Arg Cys Val Val Ile Gly Ser
    130                 135                 140
Gly Gly Ile Leu His Gly Leu Glu Leu Gly His Thr Leu Asn Gln Phe
145                 150                 155                 160
Asp Val Val Ile Arg Leu Asn Ser Ala Pro Val Glu Gly Tyr Ser Glu
                165                 170                 175
His Val Gly Asn Lys Thr Thr Ile Arg Met Thr Tyr Pro Glu Gly Ala
            180                 185                 190
Pro Leu Ser Asp Leu Glu Tyr Tyr Ser Asn Asp Leu Phe Val Ala Val
        195                 200                 205
Leu Phe Lys Ser Val Asp Phe Asn Trp Leu Gln Ala Met Val Lys Lys
    210                 215                 220
Glu Thr Leu Pro Phe Trp Val Arg Leu Phe Phe Trp Lys Gln Val Ala
225                 230                 235                 240
Glu Lys Ile Pro Leu Gln Pro Lys His Phe Arg Ile Leu Asn Pro Val
                245                 250                 255
Ile Ile Lys Glu Thr Ala Phe Asp Ile Leu Gln Tyr Ser Glu Pro Gln
            260                 265                 270
Ser Arg Phe Trp Gly Arg Asp Lys Asn Val Pro Thr Ile Gly Val Ile
        275                 280                 285
Ala Val Val Leu Ala Thr His Leu Cys Asp Glu Val Ser Leu Ala Gly
    290                 295                 300
Phe Gly Tyr Asp Leu Asn Gln Pro Arg Thr Pro Leu His Tyr Phe Asp
305                 310                 315                 320
Ser Gln Cys Met Ala Ala Met Asn Phe Gln Thr Met His Asn Val Thr
```

```
                325                 330                 335
Thr Glu Thr Lys Phe Leu Leu Lys Leu Val Lys Glu Gly Val Val Lys
            340                 345                 350
Asp Leu Ser Gly Gly Ile Asp Arg Glu Phe
        355                 360
```

<210> 2
<211> 1086
<212> DNA
<213> Homo sapience

<400> 2

```
atgagaaggc ccagcttgtt attaaaagac atcctcaaat gtacattgct tgtgtttgga     60
gtgtggatcc tttatatcct caagttaaat tatactactg aagaatgtga catgaaaaaa    120
atgcattatg tggaccctga ccatgtaaag agagctcaga aatatgctca gcaagtcttg    180
cagaaggaat gtcgtcccaa gtttgccaag acatcaatgg cgctgttatt tgagcacagg    240
tatagcgtgg acttactccc ttttgtgcag aaggccccca aagacagtga agctgagtcc    300
aagtacgatc ctccttttgg gttccggaag ttctccagta aagtccagac cctcttggaa    360
ctcttgccag agcacgacct ccctgaacac ttgaaagcca agacctgtcg gcgctgtgtg    420
gttattggaa gcggaggaat actgcacgga ttagaactgg gccacaccct gaaccagttc    480
gatgttgtga taaggttaaa cagtgcacca gttgagggat attcagaaca tgttggaaat    540
aaaactacta taaggatgac ttatccagag ggcgcaccac tgtctgacct tgaatattat    600
tccaatgact tatttgttgc tgttttattt aagagtgttg atttcaactg gcttcaagca    660
atggtaaaaa aggaaaccct gccattctgg gtacgactct tcttttggaa gcaggtggca    720
gaaaaaatcc cactgcagcc aaaacatttc aggattttga atccagttat catcaaagag    780
actgcctttg acatccttca gtactcagag cctcagtcaa ggttctgggg ccgagataag    840
aacgtcccca caatcggtgt cattgccgtt gtcttagcca cacatctgtg cgatgaagtc    900
agtttggcgg gttttggata tgacctcaat caacccagaa cacctttgca ctacttcgac    960
agtcaatgca tggctgctat gaactttcag accatgcata atgtgacaac ggaaaccaag   1020
ttcctcttaa agctggtcaa agagggagtg gtgaaagatc tcagtggagg cattgatcgt   1080
gaattt                                                              1086
```

<210> 3
<211> 21
<212> DNA
<213> Artificial Sequence

<220>
<223> Primer

<400> 3

```
taatcggaag tggcggaata c                                          21
```

<210> 4
<211> 21
<212> DNA
<213> Artificial Sequence

<220>
<223> Primer

<400> 4

```
ctcggcccca gaaccttgac c                                          21
```

<210> 5
<211> 54
<212> DNA
<213> Artificial Sequence

<220>
<223> Nucleotide Sequence for shRNA

<400> 5

```
gaagacccag cttgttaatg tgtgctgtcc attaacaagc tgggtcttct tttt    54

<210> 6
<211> 54
<212> DNA
<213> Artificial Sequence

<220>
<223> Nucleotide Sequence for shRNA

<400> 6
gccaatgatt tgttcgttag tgtgctgtcc taacgaacaa atcattggct tttt    54
```